(19) Europäisches Patentamt
European Patent Office
Office européen des brevets

(11) Numéro de publication: **0 248 703 B1**

# (12) FASCICULE DE BREVET EUROPEEN

(45) Date de publication de fascicule du brevet: **15.05.91**

(51) Int. Cl.[5]: **C07K 7/06**, A61K 37/02

(21) Numéro de dépôt: **87401142.2**

(22) Date de dépôt: **21.05.87**

(54) **Nouveaux dérivés de synergistines, leur préparation et les compositions pharmaceutiques qui les contiennent.**

(30) Priorité: **22.05.86 FR 8607270**

(43) Date de publication de la demande: **09.12.87 Bulletin 87/50**

(45) Mention de la délivrance du brevet: **15.05.91 Bulletin 91/20**

(84) Etats contractants désignés: **AT BE CH DE ES FR GB GR IT LI LU NL SE**

(56) Documents cités:
**EP-A- 135 410**
**EP-A- 0 133 096**
**EP-A- 0 133 097**
**EP-A- 0 133 098**

(73) Titulaire: **RHONE-POULENC RORER SA**
**20, avenue Raymond Aron**
**F-92160 Antony(FR)**

(72) Inventeur: **Barriere, Jean-Claude**
**23 rue Henri Gilbert**
**F-91300 Massy(FR)**
Inventeur: **Cotrel, Claude**
**17A avenue du Docteur Arnold Netter**
**F-75012 Paris(FR)**
Inventeur: **Paris, Jean-Marc**
**8 rue des Acacias**
**F-77360 Vaires sur Marne(FR)**

(74) Mandataire: **Savina, Jacques et al**
**RHONE-POULENC SANTE, Service Brevets,**
**20 Avenue Raymond Aron**
**F-92165 Antony Cédex(FR)**

**EP 0 248 703 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La demande de brevet européen EP 133097 décrit des dérivés de synergistines substitués en position 5δ qui présentent l'avantage d'être solubles.

La pristinamycine et la virginiamycine sont des produits connus : J. Preud'homme et coll., Bull. Soc. Chim. Fr., 2, 585-91 (1968).

La présente invention concerne de nouveaux dérivés de synergistines de formule générale :

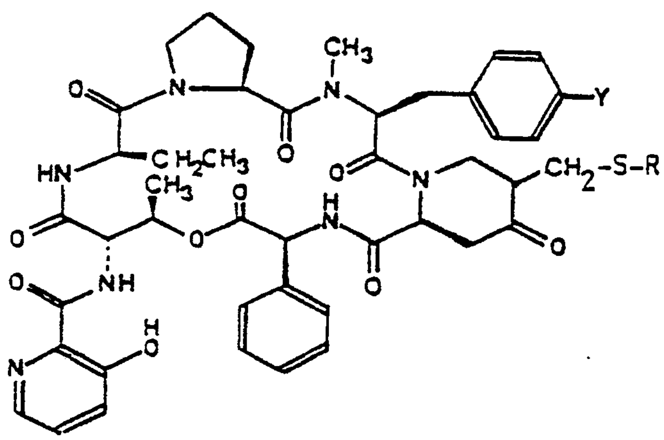

(I)

leurs sels, leurs préparation et les compositions pharmaceutiques qui les contiennent.

Dans la formule générale (I), le symbole Y représente un atome d'hydrogène ou un radical diméthylamino et le symbole R représente un radical quinuclidinyle-3 ou -4.

Il est entendu que les produits de formule générale (I) présentent des formes isomères et que ces isomères et leurs mélanges entrent dans le cadre de la présente invention.

A/ Selon l'invention, les produits de formule générale (I) dans laquelle Y et R sont définis comme précédemment, peuvent être préparés par action d'un thiol de formule générale :

R SH (II)

dans laquelle R a la définition donnée précédemment sur un produit de formule générale :

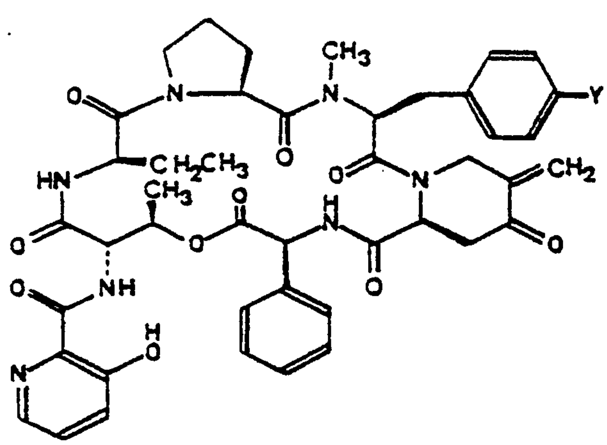

(III)

dans laquelle Y est défini comme précédemment.

On opère généralement dans un solvant organique tel qu'un alcool comme le méthanol, une cétone (acétone par exemple) ou un solvant chloré comme le chloroforme ou un mélange de ces solvants, à une température comprise entre -78 °C et la température de reflux du mélange réactionnel, de préférence à une

température voisine de 20 °C.

Lorsque le symbole R représente un radical quinuclidinyle-3, il est entendu que le thiol de configuration (R) ou (S) conduit au dérivé de synergistine de formule générale (I) de configuration correspondante.

Le thiol de formule générale (II) dans laquelle R est un radical quinuclidinyle-3 peut être obtenu à partir du thioloester de formule générale :

R-S-COR'    (IV)

dans laquelle R est défini comme précédemment et R' représente un radical alcoyle contenant 1 à 4 atomes de carbone, de préférence un radical méthyle, par toute méthode connue pour obtenir un thiol à partir d'un thioloester, sans toucher au reste de la molécule. On opère notamment par alcoolyse en milieu alcalin, par exemple dans un alcool tel que le méthanol, en présence de méthylate de sodium ou de soude, à une température comprise entre 20 °C et la température de reflux du mélange réactionnel.

Le thioloester de formule générale (IV) peut être préparé par analogie avec la méthode décrite par R.P. Volante, Tet. Let. 22 (33), 3119 (1981) à partir d'un alcool de formule générale :

R OH    (V)

dans laquelle R est défini comme précédemment et d'un acide thiocarboxylique de formule générale :

R' CO SH    (VI)

dans laquelle R' est défini comme ci-dessus, par traitement par un mélange de triphénylphosphine et d'azodicarboxylate de dialcoyle, par exemple l'azodicarboxylate de diisopropyle.

La réaction s'effectue dans des conditions analogues à celles décrites dans la référence ci-dessus.

Il est entendu que l'alcool de formule générale (V) de forme (R) ou (S) conduit respectivement au thioloester de formule générale (IV) de configuration (S) ou (R).

L'alcool de formule générale (V) de forme (R) ou (S) peut être préparé selon la méthode décrite par B. RINGDAHL et coll., Acta. Pharm. Suec., 16, 281 (1979).

Le thiol de formule générale (II) dans laquelle R est un radical quinuclidinyle-4 peut être obtenu selon la méthode décrite par A. Grob, Helv. Chim. Acta, 57, 2339 (1974).

Les produits de formule générale (III) peuvent être préparés comme décrit dans la demande de brevent européen EP 133098.

Lorsque les produits de formule générale (I) se présentent sous forme d'un mélange d'isomères, il est possible de séparer ceux-ci par toute méthode connue qui n'affecte pas le reste de la molécule ; par exemple par chromatographie liquide hautes performances.

Les nouveaux produits de formule générale (I) peuvent être purifiés par les méthodes connues habituelles telles que cristallisation, chromatographie ou extraction successives en milieu acide et basique. Pour l'homme du métier connaissant la sensibilité des synergistines en milieu alcalin, il est évident que l'on entend par "milieu basique" un milieu juste suffisamment alcalin pour libérer la substante-mère de son sel d'addition avec un acide, c'est-à-dire un milieu dont le pH n'excède pas 7,5 à 8.

Les nouveaux produits de formule générale (I) peuvent être transformés en sel d'addition avec les acides par action d'un acide dans un solvant organique tel qu'un alcool, une cétone, un ester ou un solvant chloré. Le sel précipite, éventuellement après concentration de sa solution ; il est séparé par filtration ou décantation. Les sels d'addition avec les acides peuvent également être obtenus à l'état de solutions aqueuses par addition d'une solution aqueuse d'acide correspondant sur le produit de formule générale (I).

Il est bien connu que les syergistines obtenues par fermentation constituent des produits très recherchés par les médecins pour le traitement de beaucoup d'affections dues à des bactéries Gram-positives (du genre Staphylocoques, Streptocoques, pneumocoques, entérocoques) et Gram-négatives (du genre Haemophilus, gonocoques, méningocoques). Toutefois, ces produits présentent l'inconvénient d'être insolubles en milieu aqueux et ne peuvent donc être administrés que par voie orale, généralement sous forme de gellules, de dragées ou de comprimés. Compte tenu de cette insolubilité, il est impossible

d'utiliser les synergistines connues lorsque le malade n'est pas en l'état de déglutir ; c'est notamment le cas en pédiatrie et en réanimation, alors que le spectre d'activité de ces produits en ferait une indication précieuse dans un grand nombre de circonstances, par exemple dans les cas de septicémies comateuses.

Les nouveaux produits selon l'invention présentent l'avantage considérable de pouvoir être solubilisés dans l'eau, à l'état de sels, aux doses thérapeutiquement utilisables, tout en conservant le spectre général d'activité des synergistines. Ils sont notamment actifs sur Staphylococcus aureus Smith in vitro à des concentrations comprises entre 1 et 125 µg/ml et in vivo à des doses comprises entre 5 et 50 mg/kg par voie sous-cutanée chez la souris.

De plus ils sont particulièrement intéressants du fait de leur faible toxicité.

Leur $DL_{50}$ est généralement supérieure à 150 mg/kg chez la souris par voie sous-cutanée.

Les produits selon la présente invention manifestent de plus un phénomène de synergie lorsqu'ils sont associés à la pristinamycine $II_A$ ou à un dérivé soluble de la pristinamycine $II_B$ de formule générale :

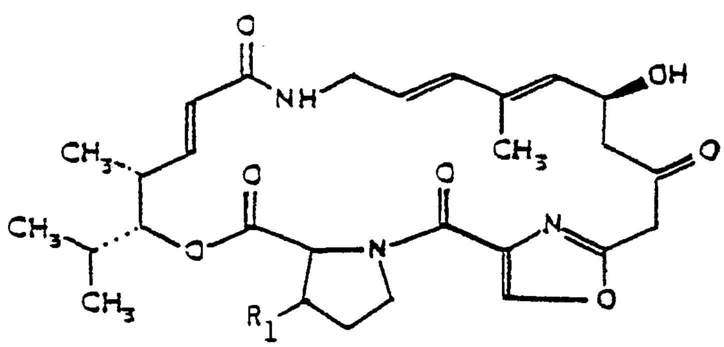

(VII)

dans laquelle $R_1$ représente

1° un radical alcolythio contenant 1 à 5 atomes de carbone, substitué

- i) par un ou deux radicaux alcoylamino ou dialcoylamino dont les parties alcoyle qui contiennent 1 à 5 atomes de carbone peuvent éventuellement former ensemble avec l'atome d'azote auquel elles sont liées un hétérocycle saturé choisi parmi pyrrolidinyle-1, pipéridino, azétidinyle-1, azépinyle-1, morpholino, thiomorpholino et pipérazinyle-1 (éventuellement substitué par un radical alcoyle contenant 1 à 5 atomes de carbone), ou bien
- ii) par un radical pyrrolidinyle-2 ou 3, pipéridyle-2, 3 ou 4, azétidinyle-2 ou 3 ou azépinyle-2, 3 ou 4,

2° un radical de formule générale :

$$Het\text{-}S\text{-} \qquad (VIII)$$

dans laquelle Het représente un radical pyrrolidinyle-3, pipéridyle-3 ou 4, azétidinyle-3 ou azépinyle-3 ou 4, éventuellement substitué par un radical alcoyle contenant 1 à 5 atomes de carbone,

3° un radical dialcoylamino dont les parties alcoyle qui contiennent 1 à 5 atomes de carbone peuvent éventuellement former ensemble avec l'atome d'azote auquel elles sont liées un hétérocycle saturé choisi parmi pyrrolidinyle-1, pipéridino, azétidinyle-1, azépinyle-1, morpholino, thiomorpholino et pipérazinyle-1 (éventuellement substitué par un radical alcoyle contenant 1 à 5 atomes de carbone),

4° un radical de formule générale :

$$R_2 - \underset{(O)_n}{S} - \qquad (IX)$$

dans laquelle $R_2$ représente

- i) soit un radical hétérocyclyle azoté de 4 à 7 chaînons, contenant éventuellement un ou plusieurs autres hétéroatomes choisis parmi l'azote, l'oxygène ou le soufre à l'état de sulfoxyde ou de sulfone, et éventuellement substitué par un radical alcoyle,
- ii) soit une chaîne alcoyle contenant 2 à 4 atomes de carbone substituée par 1 ou 2 radicaux

choisis parmi phényle, cycloalcoylamino ou N-alcoyl N-cycloalcoyl-amino contenant 3 à 6 chaînons, alcoylamino, dialcoylamino ou dialcoylcarbamoyloxy (les parties alcoyle de ces 2 derniers radicaux pouvant éventuellement former avec l'atome d'azote auquel elles sont rattachées un hétérocycle saturé ou insaturé de 4 à 7 chaînons contenant éventuellement un autre hétéroatome choisi parmi l'azote l'oxygène ou le soufre à l'état de sulfoxyde ou de sulfone, et éventuellement substitué par un radical alcoyle), ou substituée par un ou plusieurs hétérocycles azotés de 4 à 7 chaînons contenant éventuellement 1 ou 2 autres hétéroatomes choisis parmi l'azote, l'oxygène ou le soufre à l'état de sulfoxyde ou de sulfone, et éventuellement substitués par un radical alcoyle, lesdits hétérocycles étant rattachés à la chaîne qui les porte par l'intermédiaire d'un atome de carbone, étant entendu que l'un au moins de substituants portés par la chaîne alcoyle ci-dessus est un substituant azoté capable de former des sels,

- iii) soit un radical [méthyl-1 pyrrolidinyl-2 (S)] méthyle, et le symbole n est égal à 1 ou 2, étant entendu que les radicaux et portions alcoyle cités ci-dessus sont droits ou ramifiés et contiennent sauf mention spéciale 1 à 10 atomes de carbone.

Les dérivés de pristinamycine $II_B$ de formule générale (VII) peuvent présenter des formes isomères. Il est entendu que ces formes isomères aussi bien que leurs mélanges peuvent être avantageusement associés aux produits de formule générale (I).

Dans la définition de la formule générale (VII), paragraphe 4°,

- lorsque $R_2$ représente un radical hétérocycle, ce radical peut être choisi à titre d'exemple parmi : azétidinyle-3, pyrrolidinyle-3, pipéridyle-3 ou -4 azépinyle-3 ou -4,
- lorsque $R_2$ représente un radical hétérocyclylalcoyle, le radical hétérocyclyle peut être choisi à titre d'exemple parmi les radicaux cités ci-dessus ou les radicaux azétidinyle-2, pyrrolidinyle-2, pipéridyle-2, azépinyle-2, pipérazinyle, alcoyl-4 pipérazinyle, quinolyle, isoquinolyle ou imidazolyle,
- lorsque $R_2$ contient un radical dialcoylamino ou dialcoylcarbamoyloxy dont les parties alcoyle forment avec l'atome d'azote auquel elles sont rattachées un hétérocycle, ce dernier peut être choisi à titre d'exemple parmi : azétidinyle-1, pyrrolidinyle-1, pipéridino, azépinyle-1, morpholino, thiomorpholino à l'état de sulfoxyde ou de sulfone, pipérazinyle-1, alcoyl-4 pipérazinyle-1, N-alcoyl homopipérazinyle-1, imidazolyle-1.

Les produits de formule générale (VII) dans laquelle $R_1$ est défini comme précédemment en 1°, 2° et 3° font l'objet de la demande de brevet européen EP 135410 et peuvent être préparés à partir de la pristinamycine $II_A$ selon la méthode décrite dans cette demande de brevet.

Les produits de formule générale (VII) dans laquelle $R_1$ est défini comme précédemment en 4° peuvent être préparés par oxydation d'un dérivé de pristinamycine $II_B$, de son sel ou d'un dérivé protégé de formule générale :

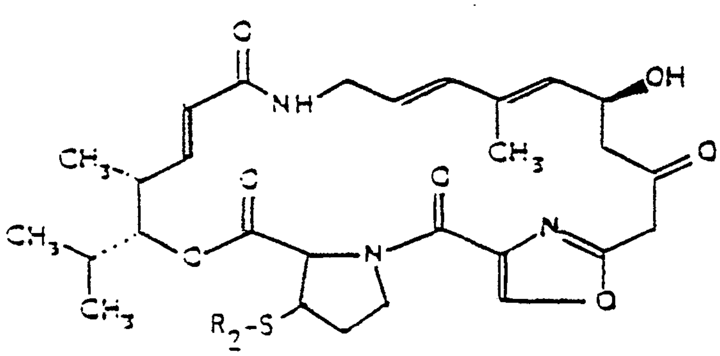

(X)

dans laquelle $R_2$ est défini comme précédemment, étant entendu que dans les cas où $R_2$ contient un hétérocycle soufré, l'atome de soufre peut se trouver à l'état de sulfure, de sulfoxyde ou de sulfone.

La réaction s'effectue généralement au moyen d'un agent d'oxydation éventuellement préparé in situ, en milieu aqueux ou dans un solvant organique, de préférence un solvant chloré (chlorure de méthylène, dichloro-1,2 éthane, chloroforme par exemple) ou un alcool (méthanol, t.butanol par exemple) ou un mélange de ces solvants. Eventuellement on opère sous azote.

Parmi les agents d'oxydation convenables pour obtenir un produit de formule générale (VII) dans laquelle n = 1, peuvent être cités les peracides organiques : acides percarboxyliques ou persulfoniques (par exemple les acides peracétique, petrifluoracétique, performique, perbenzoïque, m.chloroperbenzoïque, p.nitroperbenzoïque, permaléique, monoperphtalique, percamphorique ou p.toluènepersulfonique) ou les

peracides minéraux (par exemple acide periodique ou persulfurique).

Lorsque l'on veut obtenir un produit de formule générale (VII) dans laquelle n = 2, on opère avantageusement en présence de dioxyde de sélénium et d'eau oxygénée sur le sel du produit de formule générale (X), ou en présence d'un peracide tel que ceux cités ci-dessus notamment l'acide petrifluoracétique, ou l'acide m.chloroperbenzoïque.

Lorsque l'on met en oeuvre le dérivé de pristinamycine $II_B$ de formule générale (X) sous forme de sel, on utilise les sels formés avec les acides organiques ou minéraux, de préférence avec les acides trifluoracétique, tartrique, acétique, benzoïque ou chlorhydrique.

Lorsque le produit de formule générale (X) est utilisé sous forme de sel ou de dérivé protégé, la réaction s'effectue avantageusement à une température comprise entre -40 et 50 °C.

Lorsque l'on veut obtenir un produit de formule générale (VII) dans laquelle n = 1, il est également intéressant d'opérer à partir de dérivé de pristinamycine $II_B$ de formule générale (X) en présence d'un bicarbonate alcalin (bicarbonate de sodium par exemple) à une température comprise entre -60 et -40 °C.

Lorsque $R_2$ contient un substituant alcoylamino ou cycloalcoylamino, il est également possible de mettre en oeuvre un dérivé protégé du produit de formule générale (X), ce dernier peut être protégé par tout groupement protecteur d'amine dont la mise en place et l'élimination n'affectent pas le reste de la molécule ; on utilise avantageusement le groupement trifluoracétyle qui peut être éliminé après la réaction par traitement par un bicarbonate alcalin (bicarbonate de sodium ou de potassium) en solution aqueuse.

Les produits de formule générale (X) peuvent être préparés par action d'un thiol de formule générale :

$$R_2\text{-SH} \qquad \text{(XI)}$$

dans laquelle $R_2$ est défini comme précédemment, sur la pristinamycine $II_A$, par analogie avec la méthode décrite dans la demande de brevet européen EP 135410.

Les produits de formule générale (VII) dans laquelle n est égal à 2, peuvent également être préparés par oxydation d'un produit de formule générale (VII) dans laquelle n est égal à 1.

La réaction s'effectue dans des conditions analogues aux conditions décrites précédemment pour obtenir un produit de formule générale (VII) dans laquelle n = 2 à partir d'un dérivé de pristinamycine $II_B$ de formule générale (X).

Les produits de formule générale (XI) peuvent être préparés selon ou par analogie avec les méthodes décrites ci-après dans les exemples et notamment selon :

- G.G. Urquart et coll., Org. Synth., 21, 36 (1941)
- A.I. Vogel, J. Chem. Soc., 1822 (1948)
- J.H. Chapman et L.N. Owen, J. Chem. Soc., 579 (1950)
- H.R. Snyder et coll., J. Am. Chem. Soc., 69, 2672 (1947)
- D.D. Reynolds et coll., J. Org. Chem., 26, 5125 (1961)
- J.W. Haeffele et coll., Proc. Sci. Toilet Goods Assoc., 32, 52(1959)
- H. Barrer et coll., J. Org. Chem., 27, 641 (1962)
- J.H. Biel et coll., J. Amer. Chem. Soc., 77, 2250 (1955).

Le cas échéant les isomères des produits de formule générale (VII) peuvent être séparés par chromatographie ou par chromatographie liquide hautes performances.

Les dérivés de synergistines selon la présente invention synergisent l'action antibactérienne de la pristinamycine $II_A$ sur Staphylococcus aureus Smith in vitro à des doses comprises entre 0,1 et 10 µg/cm3 et in vivo chez la souris à des doses comprises entre 10 et 200 mg/kg par voie sous-cutanée, lorsqu'ils sont associés dans des proportions variant entre 10-90 % et 90-10 %.

Pour l'emploi thérapeutique, il peut être fait usage des nouveaux produits selon l'invention tels quels, c'est-à-dire à l'état de base, mais pour l'emploi en solution aqueuse, ce qui constitue l'avantage principal des produits selon l'invention, il est particulièrement avantageux de faire usage de leurs sels pharmaceutiquement acceptables, c'est-à-dire de sels non toxiques aux doses d'utilisation, en association avec la pristinamycine $II_A$ ou avec un dérivé soluble de la pristinamycine $II_B$ de formule générale (VII) lui-même solubilisé soit à l'état de sel pharmaceutiquement acceptable, soit le cas échéant, à l'état de base lorsque la solubilité est suffisante pour que la solution obtenue contienne une quantité de produit au moins égale à la dose thérapeutiquement active.

Comme sels pharmaceutiquement acceptables, on peut citer les sels d'addition avec les acides minéraux tels que chlorhydrates, bromhydrates, sulfates, nitrates, phosphates, ou organiques tels que acétates, propionates, succinates, maléates, fumarates, méthanesulfonates, p.toluènesulfonates, iséthionates, citrates, adipates, lactobionates ou des dérivés de substitution de ces composés.

Les exemples suivants montrent comment l'invention peut être mise en pratique. Les spectres de RMN des produits illustrés dans ces exemples et dans les exemples de référence présentent des caractéristiques générales qui sont communes à tous les produits de formule générale (I) ou de formule générale (VII) et des caractéristiques particulières qui sont propres à chacun des produits en fonction des substituants.

Dans les exemples suivants ne sont mentionnées que les caractéristiques particulières dues aux radicaux variables. Tous les protons sont désignés selon la numérotation indiquée dans la formule générale (XII) et recommandée par J.O. ANTEUNIS et al [Eur. J. Biochim., 58, 259 (1975)].

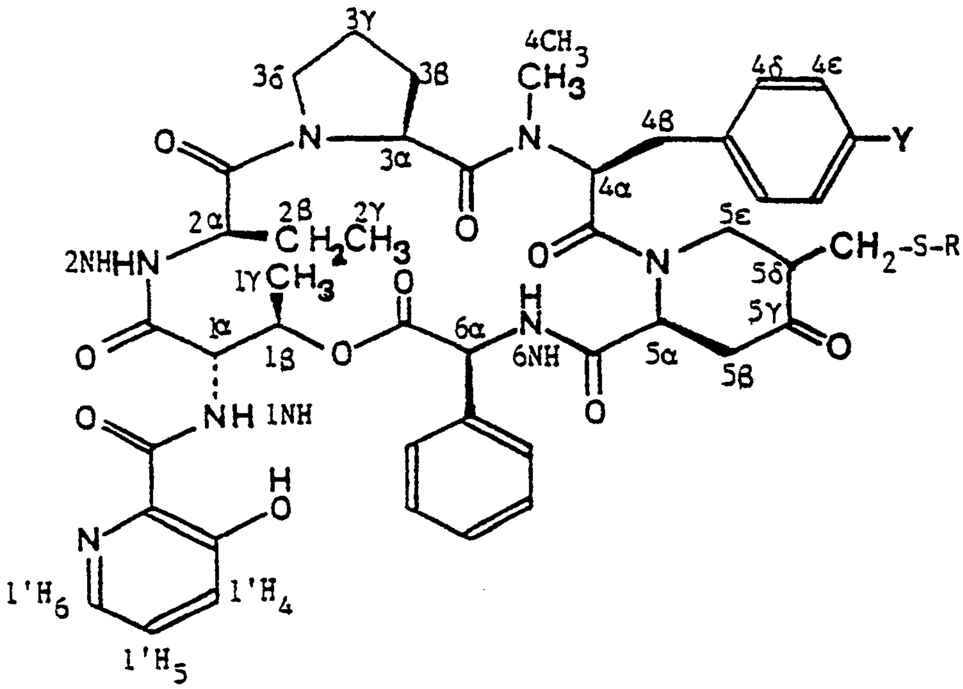

(XII)

Pour les produits de formule générale (VII) tous les protons sont désignés selon la numérotation indiquée dans la formule suivante :

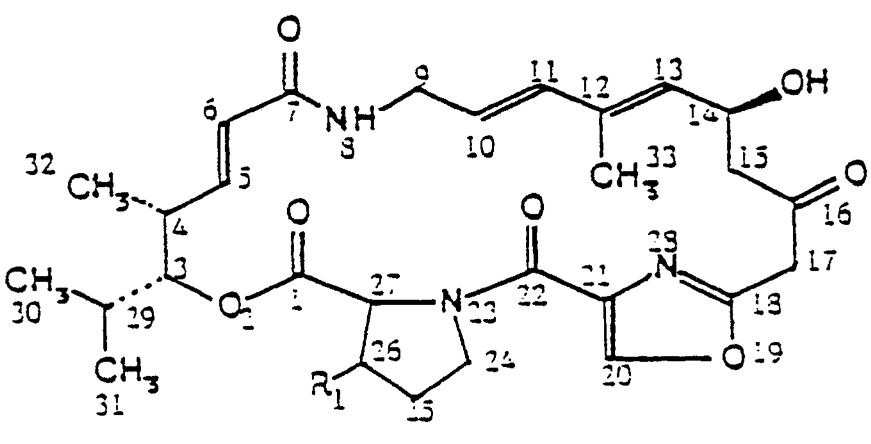

(XIII)

Sauf mention spéciale les spectres ont été faits à 250 MHz dans le deutérochloroforme ; les déplacements chimiques sont exprimés en ppm par rapport au signal du tétraméthylsilane. Les abréviations utilisées dans la suite sont les suivantes :

s = singulet
d = doublet
t = triplet
mt = multiplet
m = massif
dd = doublet de doublet
dt = doublet de triplet
ddd = doublet de doublet de doublet
dddd = doublet de doublet de doublet de doublet

Il est entendu que les différents isomères ont été classés arbitrairement selon les déplacements chimiques observés en RMN.

On appelle isomère $A_1$ et isomère $A_2$ des produits de formule générale (XIII) dans laquelle $R_1$ est un radical $R_2S(O)_n$- pour lequel n = 1, les isomères qui présentent les caractéristiques : environ 1,7 (s, $-CH_3$ en 33) ; environ 3,8

(s, $>CH_2$ en 17) ;

<5 (d, $H_{27}$) isomère $A_2$ ou >5 (d, $H_{27}$) isomère $A_1$ ; environ 5,50 (d large, $H_{13}$) ; environ 6,20 (d, $H_{11}$) ; environ 6,6

($>NH$ en 8) ;

≧8 (s, $H_{20}$).

On appelle isomère $B_1$ et isomère $B_2$ des produits de formule générale (XIII) dans laquelle $R_1$ est un radical $R_2S(O)_n$ - pour lequel n = 1, les isomères qui présentent les caractéristiques : environ 1,5 (s, $-CH_3$ en 33) ; environ 3,7 et 3,9

(2d, $>CH_2$ en 17) ;

environ 4,8 (mt, $H_{13}$) ; <5 (d, $H_{27}$) isomère $B_2$ ou >5 (d, $H_{27}$) isomère $B_1$ ; environ 5,70 (AB limite, $H_{11}$ et $H_{10}$) ; environ 7,7

($>NH$ en 8) ;

environ 7,8 (s, $H_{20}$).

On appelle isomère A du produit de formule générale (XIII) dans laquelle $R_1$ est autre que $R_2S(O)_n$-, l'isomère qui présente des caractéristiques RMN identiques à celles énoncées ci-dessous pour les isomères $A_1$ et $A_2$ des produits de formule générale (XIII), dans laquelle $R_1$ est un radical $R_2S(O)_n$-, étant entendu que l'H en 27 est caractérisé par : 4,7 environ (d, J ≦ 1 Hz).

On appelle isomère B du produit de formule générale (XIII) dans laquelle $R_1$ est autre que $R_2S(O)_n$-, l'isomère qui présente des caractéristiques RMN identiques à celles énoncées ci-dessus pour les isomères $B_1$ et $B_2$ des produits de formule générale (XIII), étant entendu que l'H en 27 est caractérisé par : 4,6 environ (d, J ≧ 2,5 Hz).

Dans les exemples qui suivent, on appelle chromatographie "flash" une technique de purification caractérisée en ce que l'on opère sous une pression moyenne (50 kPa) en utilisant une silice de granulométrie 40-53 µm, selon W.C. STILL, M. KAHN et A, MITRA [J. Org. Chem., 43, 2923 (1978)].

Dans les exemples décrits ci-après, sauf mention spéciale, tous les produits peuvent être mis en solution aqueuse à 2 % au moins, à l'état de chlorhydrate.

EXEMPLE 1

A une solution de 4,4 g de méthylène-5δ pristinamycine $I_A$ dans un mélange de 40 cm3 de méthanol et de 20 cm3 de chloroforme, on ajoute 2,8 g de mercapto-3 quinuclidine, puis on agite la solution obtenue pendant 44 heures à une température voisine de 20 °C. Le mélange réactionnel est ensuite concentré à sec sous pression réduite (2,7 kPa) à 30 °C. Le résidu obtenu est mis en suspension dans 100 cm3 d'éther éthylique puis séparé par filtration. Le solide ainsi obtenu est lavé par 3 fois 10 cm3 d'éther éthylique puis purifié par chromatographie "flash" [éluant : chlorure de méthylène-méthanol (90/10 en volumes)] en recueillant des fractions de 100 cm3. Les fractions 11 à 35 sont réunies puis concentrées à sec sous pression réduite (2,7 kPa) à 30 °C. Le résidu est agité dans 120 cm3 d'éther éthylique. Le solide obtenu est séparé par filtration puis de nouveau purifié par chromatographie "flash" [éluant : chlorure de méthylène-méthanol (85-15 en volumes)] en recueillant des fractions de 100 cm3. Les fractions 3 à 7 sont réunies puis concentrées à sec sous pression réduite (2,7 kPa) à 30 °C. Le résidu est agité dans 50 cm3 d'éther éthylique et le solide ainsi obtenu est séparé par filtration, lavé avec 3 fois 5 cm3 d'éther éthylique, puis séché sous pression réduite (27 Pa) à 20 °C. On obtient ainsi 1,7 g (quinuclidinyl-3) thiométhyl-5δ pristinamycine $I_A$ sous forme d'un solide jaune pâle fondant vers 200 °C.

Spectre RMN
Mélange de 4 isomères
2,88 et 2,89 (2s, 4 $-N(CH_3)_2$)
3,21 et 3,22 (2s, 4 $-CH_3$)
6,51 et 6,53 (2d, 2 -NH-)
6,57 et 6,58 (2d, 4ε)
7,82 et 7,85 (mt, l'$H_6$ des 2 isomères majoritaires)
7,95 (mt, l'$H_6$ des 2 isomères minoritaires)
8,78 et 8,81 (2d, 6 -NH- des 2 isomères majoritaires)
8,98 et 9,0 (2d, 6 -NH- des 2 isomères minoritaires)

On obtient une solution aqueuse à 5 % de (quinuclidinyl-3) thiométhyl-5δ pristinamycine $I_A$ , avec :

| | |
|---|---|
| produit ............................ | 100 mg |
| acide chlorhydrique 0,1N ........... | 0,98 cm3 |
| eau distillée .................. | qsp 2 cm3 |

La mercapto-3 quinuclidine peut être préparée de la manière suivante :

A une solution de 14,5 g d'acétylthio-3 quinuclidine dans 150 cm3 de méthanol, on ajoute 0,5 g de méthylate de sodium. Le mélange réactionnel est alors chauffé à reflux pendant 1 heure. On ajoute à nouveau 0,5 g de méthylate de sodium puis on chauffe à reflux pendant 2 heures. Le mélange réactionnel est concentré à sec sous pression réduite (2,7 kPa) à 40 °C. Au résidu obtenu, on ajoute 40 cm3 d'eau distillée puis environ 1 cm3 d'acide acétique pour obtenir un pH voisin de 8. Le mélange obtenu est extrait par 3 fois 20 cm3 de chlorure de méthylène. Les phases organiques réunies sont séchées sur du sulfate de sodium, filtrées puis concentrées à sec sous pression réduite (2,7 kPa) à 30 °C. L'huile brune obtenue est distillée sous pression réduite (920 Pa), la fraction distillant vers 94 °C est recueillie. On obtient ainsi 2,8 g de mercapto-3 quinuclidine.

L'acétylthio-3 quinuclidine peut être obtenue de la manière suivante :

A une solution maintenue à 5 °C sous atmosphère d'azote de 42 g de triphénylphosphine dans 300 cm3 de tétrahydrofuranne, on ajoute goutte à goutte en 30 minutes, 31,6 cm3 d'azodicarboxylate de diisopropyle. On agite ensuite la suspension obtenue pendant 30 minutes à 5 °C. On ajoute ensuite à cette suspension maintenue à 5 °C, en 30 minutes, une solution de 10,2 g d'hydroxy-3 quinuclidine et de 11,4 cm3 d'acide thiolacétique dans 600 cm3 de tétrahydrofuranne. Le mélange réactionnel est agité ensuite pendant 20 heures à une température voisine de 20 °C. Il est alors concentré à sec sous pression réduite (2,7 kPa) à 40 °C. L'huile obtenue est dissoute dans 400 cm3 d'éther éthylique, puis lavée par 3 fois 160 cm3 d'acide chlorhydrique. Les phases aqueuses réunies sont lavées par 100 cm3 d'éther éthylique, puis neutralisées à pH voisin de 7 par addition de bicarbonate de sodium. Le pH de la solution obtenue est ensuite ajusté vers 9 par addition de quelques gouttes d'une solution aqueuse de soude 10N. On extrait par 3 fois 200 cm3 de chlorure de méthylène. Les phases organiques réunies sont séchées sur du sulfate de sodium, filtrées et concentrées à sec sous pression réduite (2,7 kPa) à 30 °C. On obtient ainsi 14,7 g d'acétylthio-3 quinuclidine sous forme d'une huile brune [Rf = 0,33 ; éluant : chlorure de méthylène-méthanol (90-10 en volumes)].

EXEMPLE 2

En opérant d'une manière analogue à celle décrite à l'exemple 1, mais à partir de 6,15 g de méthylène-5δ prisinamycine $I_A$ et de 1,1 g de mercapto-3 quinuclidine(S) et après deux purifications par chromatographie "flash" en recueillant des fractions de 50 cm3 [1ière chromatographie "flash" : éluant : chlorure de méthylène-méthanol (90-10 en volumes), concentration à sec des fractions 12 à 36 ; 2ème chromatographie "flash" : éluant : chlorure de méthylène-méthanol (90-10 en volumes), concentration à sec des fractions 3 à 20], on obtient 2,6 g de [quinuclidinyl-3(S)] thiométhyl-5δ pristinamycine $I_A$ sous forme d'une poudre jaune pâle. Ce produit peut être obtenu à l'état cristallisé de la manière suivante :

2,6 g de [quinuclidinyl-3(S)] thiométhyl-5δ pristinamycine $I_A$ sont dissous dans 20 cm3 de méthanol. On obtient une solution jaune. Un précipité cristallin apparaît après amorçage par grattage, après filtration et séchage sous pression réduite (27 Pa) à 30 °C. On obtient 1,2 g de [quinuclidinyl-3(S)] thiométhyl-5δ pristinamycine $I_A$ sous forme de cristaux blancs fondant vers 200 °C (produit cristallisé en association avec le méthanol).

Spectre RMN ; 1 isomère (traces de l'isomère au niveau du carbone 5δ)

0,62 (dd, J = 15 et 6, 1H, $5\beta_2$ )

1,6 à 2,30 (mt, 6H, 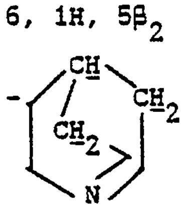 et 5δ)

2,4 (d, J = 15, 1H, $5\beta_1$ )

2,5 à 2,75 (mt, $5\varepsilon_2$ , 1H de 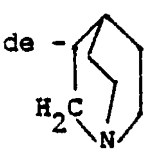 et 1H de $-CH_2$ S-)

2,9 à 3,20 (mt, -S-CH< , 1H de $-CH_2S-$ et 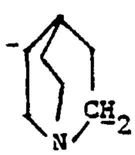 )

3,30 (mt, 1H de 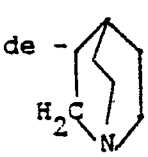 )

4,98 (dd, J = 14 et 7,5, 1H, $5\varepsilon_1$ )

5,30 (mt, 2H, 5α et 4α)

7,90 (dd, 1H, $1'H_6$)

La [quinuclidinyl-3(S) thiométhyl]-5δ pristinamycine $I_A$ peut être également recristallisée de la manière suivante :

On dissout 17,4 g de [quinuclidinyl-3(S) thiométhyl]-5δ pristinamycine $I_A$ dans 87 cm3 d'acétone préalablement portée au reflux. La solution obtenue est filtrée puis l'insoluble est rincé par 10 cm3

d'acétone. Après 3 heures à 20 °C les cristaux obtenus sont filtrés puis séchés. La recristallisation des 14,8 g obtenus dans 75 cm3 d'acétone dans les mêmes conditions donne après filtration puis séchage sous pression réduite (90 Pa) à 20 °C, 12,2 g de cristaux blancs fondant vers 185-190 °C.

La mercapto-3 quinuclidine(S) peut être obtenue de la manière suivante :

A une solution de 29 g d'acétylthio-3 quinuclidine(S) dans 30 cm3 de méthanol, maintenue à environ 25 °C, on ajoute lentement 30 cm3 d'une solution aqueuse de soude 10N. Le mélange réactionnel est agité 2 heures à une température voisine de 20 °C. Le pH du mélange réactionnel est ensuite amené à une valeur voisine de 9 par addition d'environ 10 cm3 d'acide acétique. Le mélange obtenu est extrait part 3 fois 100 cm3 de chlorure de méthylène. Les phases organiques réunies sont séchées sur du sulfate de sodium, filtrées et concentrées à sec sous pression réduite (2,7 kPa) à 20 °C. Le résidu obtenu est purifié par distillation sous pression réduite (970 Pa). On obtient ainsi 12 g de mercapto-3 quinuclidine(S) sous forme de cristaux blancs fondant à 46 °C et distillant à 95 °C sous 970 Pa ($\alpha_D^{20}$ = -118 °, C = 1,1, méthanol).

L'acétylthio-3 quinuclidine(S) peut être obtenue de manière analogue à celle décrite à l'exemple 1, mais à partir de 104,8 g de triphénylphosphine, de 80,8 g d'azodicarboxylate de diisopropyle et de 25,7 g d'hydroxy-3 quinuclidine(R). On obtient ainsi 30 g d'acétylthio-3 quinuclidine(S) sous forme d'une huile jaune. [Rf = 0,2 ; éluant : chlorure de méthylène-méthanol (90-10 en volumes)]. Selon R.P. Volante, Tet. Let., 22 (33), 3119 (1981) le carbone 3 de configuration (R) est transformé en carbone de configuration (S) durant la réaction.

L'hydroxy-3 quinuclidine(R) est préparée selon la méthode décrite par B. RINGDAHL, B. RESUL et R. DAHLBOM, Acta Pharm. Suec. 16, 281 (1979).

EXEMPLE 3

En opérant d'une manière analogue à celle décrite à l'exemple 1, mais à partir de 6,15 g de méthylène-5δ pristinamycine $I_A$ et de 1 g de mercapto-3 quinuclidine(R), et après une purification par chromatographie "flash" en recueillant des fractions de 40 cm3 [éluant : chlorure de méthylène-méthanol (85-15 en volumes)], et concentration à sec des fractions 20 à 30, on obtient 2 g de [quinuclidinyl-3(R)] thiométhyl-5δ pristinamycine $I_A$ sous forme d'une poudre beige fondant vers 200 °C.

**Spectre RMN**

0,58 (dd, J = 15 et 5,5, 1H, $5\beta_2$ )

1,5 à 2,2 (m, -S 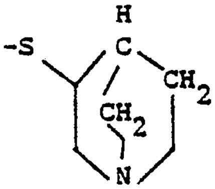 )

2,30 (m, 1H, $5\delta$)

2,35 (d, J = 15, 1H, $5\beta_1$ )

2,50 (dd, 1H de $-CH_2S-$)

2,60 (dd, 1H, $5\varepsilon_2$ )

2,78 (m, 1H de -S 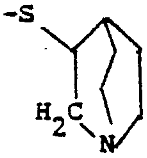 )

2,90 à 3,10 (m, 1H de $-CH_2$-S- et -S 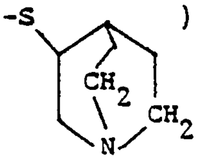 )

3,15 (m, 1H, -S-CH 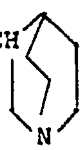 )

3,48 (m, 1H de -S 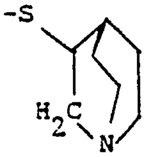 )

4,95 (dd, 1H, $5\varepsilon_1$ )

5,28 (m, 2H, $5\alpha$ et $4\alpha$)

7,87 (m, 1H x 0,85, $1'H_6$ du 1er isomère)

7,93 (m, 1H x 0,15, $1'H_6$ du 2ème isomère)

La [quinuclidinyl-3(R) thiométhyl]-5δ pristinamycine $I_A$ peut être recristallisée de la manière suivante :

On dissout 14,15 g de [quinuclidinyl-3(R) thiométhyl]-5δ pristinamycine $I_A$ dans 75 cm3 de méthanol. On ajoute à cette solution 4 cm3 d'eau distillée puis on laisse cristalliser à 4° C. Les cristaux obtenus sont filtrés puis rincés par 4 fois 10 cm3 d'un mélange méthanol-eau (95-5 en volumes). Après séchage sous pression réduite (90 Pa) à 42° C on obtient 10,22 g de cristaux blancs fondant vers 190° C.

La mercapto-3 quinuclidine(R) peut être obtenue d'une manière analogue à celle décrite à l'exemple 2, mais à partir de 32,5 g d'acétylthio-3 quinuclidine(R) et de 35 cm3 d'une solution aqueuse de soude 10N, on obtient 11,5 g de mercapto-3 quinuclidine(R) sous forme de cristaux blancs fondant à 45° C et distillant

à 90°C sous 830 Pa ($\alpha_D^{20}$ = +121°, C = 1,1, méthanol).

L'acétylthio-3 quinuclidine(R) peut être obtenue de manière analogue à celle décrite à l'exemple 1, mais à partir de 104,8 g de triphénylphosphine, de 80,8 g d'azodicarboxylate de diisopropyle et de 25,7 g d'hydroxy-3 quinuclidine(S). On obtient ainsi 33,8 g d'acétylthio-3 quinuclidine(R) sous forme d'une huile brun-clair [Rf = 0,4 ; éluant : chlorure de méthylène-méthanol (80-20 en volumes)].

L'hydroxy-3 quinuclidine(S) est préparée selon la méthode décrite par B. RINGDAHL et coll., Acta Pharm. Suec. 16, 281 (1979).

EXEMPLE 4

En opérant d'une manière analogue à celle décrite à l'exemple 1, mais à partir de 3,5 g de méthylène-5δ pristinamycine $I_A$ et de 0,6 de mercapto-4 quinuclidine et après concentration à sec on obtient un solide qui est agité dans l'éther éthylique. Après filtration on isole 3,6 g d'un solide beige qui est purifié par chromatographie "flash" en recueillant des fractions de 50 cm3 [éluant : chlorure de méthylène-méthanol (85-15 en volumes)]. Après concentration à sec des fractions 19 à 35, lavage à l'éther éthylique, filtration puis séchage du solide résultant sous pression réduite (2,7 kPa) à 20°C, on obtient 1,2 g de (quinuclidinyl-4) thiométhyl-5δ pristinamycine $I_A$ sous forme d'une poudre blanc cassé fondant vers 200°C.

Spectre RMN (2 isomères au niveau du carbone 5δ dans les proportions 85-15 environ) :

0,62 (dd, J = 15 et 5,5, 1H, $5\beta_2$ )

1,87 (t, 6H, -S- 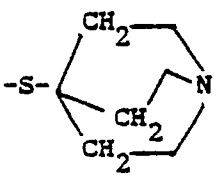 )

2,20 (m, 1H, 5δ)

2,28 (m, 1H, $-CH_2-S-$)

2,35 (d, J = 15, 1H, $5\beta_1$ )

2,47 (dd, 1H, $5\varepsilon_2$ )

3,10 (t, 6H, -S- 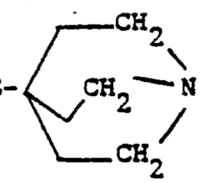 )

3,22 (dd, 1H, $-CH_2-S-$)

5,01 (dd, 1H, $5\varepsilon_1$ )

5,29 (d large, J = 5,5, 5α)

7,86 (m, 0,85H, l'$H_6$ de l'isomère majoritaire)

7,92 (m, 0,15H, l'$H_6$ de l'isomère minoritaire)

La mercapto-4 quinuclidine peut être obtenue selon la méthode décrite par A. GROB, Helv. Chim. Acta, 57, 2339 (1974).

EXEMPLE 5

On opère d'une manière analogue à celle décrite à l'exemple 1, mais à partir de 1,2 g de méthylène-5δ

virginiamycine S dans 20 cm3 de méthanol et de 0,21 g de mercapto-3 quinuclidine(R). Après purification par chromatographie "flash" en recueillant des fractions de 10 cm3 [éluant : chlorure de méthylène-méthanol (95-5 en volumes) jusqu'à la fraction 35 puis chlorure de méthylène-méthanol (80-20 en volumes)-], concentration à sec des fractions 47 à 55 et séchage sous pression réduite (2,7 kPa) à 30° C, on obtient 0,6 g de [quinuclidinyl-3 (R)] thiométhyl-5δ virginiamycine S sous forme d'une poudre blanc cassé fondant vers 185° C.

Spectre RMN (2 isomères au niveau du carbone 5δ dans les proportions 80-20 environ) :

0,4 (dd, J = 15 et 5,5, 1H, $5\beta_2$ )

1,5 à 2,2 (m, 5H, 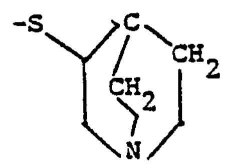 , 5δ)

2 (m, 1H, 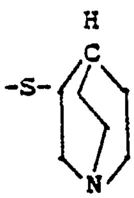 )

2,34 (d, J = 15, 1H, $5\beta_1$ )

2,52 (dd, 1H de $-CH_2S-$)

2,63 (dd, 1H, $5\varepsilon_2$ )

2,78 (dd, 1H de 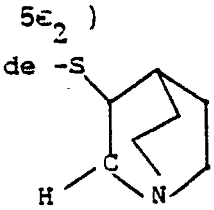 )

2,85 à 3,15 (m, 1H de $-CH_2-S-$ et 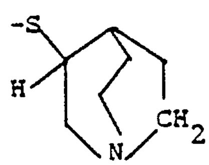 )

3,48 (m, 1H, 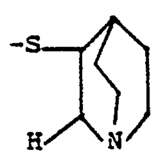 )

4,94 (dd, 1H, $5\varepsilon_1$ )

5,27 (d large, J = 5, 1H, 5α)

7,82 (dd, J = 4 et 1, 1'$H_6$ du 1er isomère)

7,9 (dd, J = 4 et 1, 1'$H_6$ du 2ème isomère)

EXEMPLE 6

On opère d'une manière analogue à celle décrite à l'exemple 1, mais à partir de 1,1 g de méthylène-5δ virginiamycine S dans 20 cm3 de méthanol et de 0,19 g de mercapto-3 quinuclidine(S). Après purification par chromatographie "flash" en recueillant des fractions de 10 cm3 [éluant : chlorure de méthylène-méthanol (90-10 en volumes)], concentration à sec des fractions 19 à 32 et séchage sous pression réduite (2,7 kPa) à 30° C, on obtient 0,5 g de [quinuclidinyl-3 (S)] thiométhyl-5δ virginiamycine S sous forme d'une poudre jaune pâle fondant vers 190° C.

Spectre RMN (2 isomères au niveau du carbone 5δ dans les proportions 85-15 environ) :

0,39 (dd, J = 15 et 5, 1H, $5\beta_2$ )

1,5 à 2,3 (m, 5H, 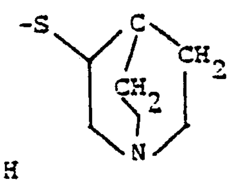 , 5δ)

2,11 (m, 1H, 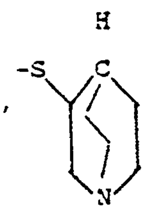 )

2,34 (d, J = 15, 1H, $5\beta_1$ )

2,52 (dd, 1H de $-CH_2S-$)

2,64 (dd, 1H, $5\varepsilon_2$ )

2,66 (dd, 1H de -S 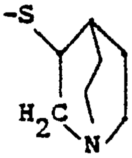 )

2,90 à 3,2 (m, 6H, 1H de $-CH_2-S-$, et -S 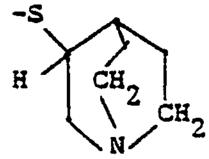 )

3,3 à 3,5 (m, 1H de -S 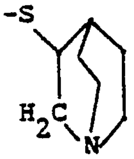 )

4,95 (dd, 1H, $5\epsilon_1$ )

5,27 (d large, 1H, 5α)

7,80 (dd, J = 4 et 1, 1H x 0,85, l'$H_6$ du 1er isomère)

7,90 (dd, J = 4 et 1, 1H x 0,15, l'$H_6$ du 2ème isomère)

EXEMPLE 7

En opérant d'une manière analogue à celle décrite à l'exemple 2 mais à partir de 1,62 g de mercapto-3 quinuclidine(S) et en agitant à -20 °C pendant 20 heures, on obtient après concentration à sec sous pression réduite (2,7 kPa) à 30 °C, 11,4 g d'une meringue beige qui est agitée dans 100 cm3 d'éther diéthylique, filtrée puis rincée par 3 fois 20 cm3 du même solvant. Ce produit peut être recristallisé dans l'acétone comme décrit à l'exemple 2 pour donner 6,6 g de [quinuclidinyl-3(S)] thiométhyl-5δ pristinamycine $I_A$ sous forme de cristaux blancs fondant vers 198-200 °C dont les caractéristiques sont identiques à celles du produit obtenu à l'exemple 2 et contenant 3 % d'isomère minoritaire dosé par HPLC.

EXEMPLE 8

A une solution de 1 g de méthylène-5δ pristinamycine $I_A$ dans 20 cm3 d'acétone on ajoute à -20 °C en 1 heure, 0,16 g de mercapto-3 quinuclidine(S) en solution dans 5 cm3 d'acétone. Après 18 heures d'agitation à -20 °C, on filtre le mélange réactionnel, on rince le solide avec 3 fois 2 cm3 d'acétone. Après séchage à l'air on obtient 0,6 g de [quinuclidinyl-3(S)] thiométhyl-5δ pristinamycine $I_A$ sous forme de cristaux blancs fondant vers 190 °C dont les caractéristiques sont identiques à celles du produit obtenu à l'exemple 2 et contenant 3 % de l'isomère minoritaire en 5δ dosé par HPLC.

EXEMPLE 9

A une solution de 1,22 g de méthylène-5δ pristinamycine $I_A$ dans 15 cm3 d'acétone, on ajoute à -78 °C, 0,16 g de mercapto-3 quinuclidine(S) en solution dans 5 cm3 d'acétone puis on agite sous azote la solution obtenue, pendant 24 heures, à -78 °C. Le mélange réactionnel est ensuite concentré à sec sous pression réduite (2,7 kPa) à 30 °C pour donner 1,4 g d'un solide blanc crème contenant 5 % d'isomère minoritaire dosé par HPLC et ayant des caractéristiques identiques à celles du produit obtenu à l'exemple 2.

EXEMPLE DE REFERENCE 1

A 3,59 g de (diisopropylamino-2 éthyl) thio-26 pristinamycine $II_B$ (isomère A) en solution dans 40 cm3 de dichlorométhane, on ajoute à 0 °C sous atmosphère d'azote, 0,4 cm3 d'acide trifluoracétique puis 1,06 g d'acide métachloroperbenzoïque à 85 % en maintenant la température à 0 °C. Après 20 heures d'agitation à 25 °C, le mélange réactionnel est additionné à une solution aqueuse saturée de bicarbonate de sodium. La phase organique est décantée puis la phase aqueuse lavée avec 3 fois 100 cm3 de chlorure de méthylène. Les phases organiques sont réunies, séchées sur du sulfate de magnésium, filtrées puis concentrées à sec sous pression réduite (2,7 kPa) à 30 °C pour donner 4,2 g d'un solide jaune qui est purifié par chromatographie "flash" [éluant : chloroforme-méthanol 90-10 en volumes)] en recueillant des fractions de 20 cm3. Les fractions 22 à 28 sont réunies et concentrées à sec sous pression réduite (2,7 kPa) à 30 °C pour donner un solide jaune clair qui est agité dans 10 cm3 d'éther éthylique. Le solide obtenu est séparé par filtration pour donner 0,62 g de (diisopropylamino-2 éthyl)sulfinyl-26 pristinamycine $II_B$ (isomère $A_2$) sous forme d'une poudre jaune fondant vers 155 °C.

Spectre RMN :

0,90 à 1,15 [mt, $-CH_3$ en 32, 31, 30, $>N(C\underline{H}(CH_3)_2)_2$ ]

1,76 (s, $-CH_3$ en 33)

2,75 à 3,15 (mt, $>CH_2$ en 15, $-H_4$ et $-S(\rightarrow O)-C\underline{H}_2-C\underline{H}_2N(C\underline{H}-)_2$ )

3,81 (s, $>CH_2$ en 17)

4,76 (d, $-H_{27}$)

5,51 (d, $-H_{13}$)

6,20 (d, $-H_{11}$)

6,48 (m, $>NH$ en 8)

8,13 (s, $-H_{20}$)

Les fractions 35 à 45 sont réunies et concentrées à sec sous pression réduite (2,7 kPa) à 30 °C pour donner un solide jaune clair qui est agité dans 15 cm3 d'éther éthylique. Le solide obtenu est séparé par filtration pour donner 1,07 g de (diisopropylamino-2 éthyl)sulfinyl-26 pristinamycine $II_B$ (isomère $A_1$ 80 %, isomère $A_2$ 20 %) sous forme d'une poudre jaune clair fondant vers 145 °C.

Spectre RMN (isomère $A_1$) :

1,72 (s, $-CH_3$ en 33)

2,70 à 3,15 (mt, $>CH_2$ en 15, $-H_4$, $-S(\rightarrow O)-CH_2-CH_2-N(CH<)-CH<$ )

3,81 (s, $>CH_2$ en 17)

5,26 (d, $-H_{27}$)

5,46 (d, $-H_{13}$)

6,15 (d, $-H_{11}$)

8,11 (s, $-H_{20}$)

La (diisopropylamino-2 éthyl) thio-26 pristinamycine $II_B$ peut être préparée de la manière suivante :

A 52 g de pristinamycine $II_A$ en solution dans un mélange de 260 cm3 de dichlorométhane et 520 cm3 de méthanol on ajoute goutte à goutte sous atmosphère d'azote à -30 °C, 16 g de diisopropylamino-2

éthanethiol en solution dans 30 cm3 de dichlorométhane. La solution est agitée pendant 20 heures à -20° C puis concentrée sous pression réduite (2,7 kPa) à 30° C. Le solide obtenu est agité avec 2 fois 1000 cm3 d'éther éthylique, séparé par filtration puis cristallisé dans 100 cm3 d'acétonitrile. Les cristaux sont séparés par filtration puis séchés sous pression réduite (90 Pa) à 40° C. On obtient ainsi 33,6 g de (diisopropylamino-2 éthyl) thio-26 pristinamycine $II_B$ (isomère A) sous forme de cristaux blancs fondant vers 122° C.

**Spectre RMN :**

1 à 1,15 (mt, $-CH_3$ isopropyl)

1,72 (s, $-CH_3$ en 33)

1,80 à 2,2(

2,50 à 3 (mt, $-SCH_2CH_2-$ 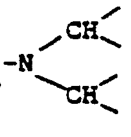 )

3,40 (d large, $-H_{26}$)

4,74 (s large, $-H_{27}$)

6,32 (m, $-NH_8$)

8,15 (s, $-H_{20}$)

Le diisopropylamino-2 éthanethiol peut être préparé selon la méthode décrite par D.D. REYNOLDS, D.L. FIELDS et D.L. JOHNSON, J. Org. Chem. 26, 5125 (1961).

EXEMPLE DE REFERENCE 2

A 10 g de (diisopropylamino-2 éthyl) thio-26 pristinamycine $II_B$ (isomère A) en solution dans 300 cm3 de chloroforme on ajoute 1,22 g de bicarbonate de sodium. On refroidit à -50° C et on ajoute goutte à goutte 2,98 g d'acide métachloroperbenzoïque à 98 % en solution dans 100 cm3 de chloroforme. Le mélange est agité 2 heures 15 minutes à -50° C puis additionné d'une solution aqueuse saturée de bicarbonate de sodium. Après 15 minutes d'agitation à 25° C, le mélange est décanté puis la phase aqueuse est lavée avec 3 fois 200 cm3 de dichlorcméthane. Les phases organiques sont rassemblées, séchées sur sulfate de magnésium, filtrées puis concentrées à sec sous pression réduite (2,7 kPa) à 30° C pour donner 10,62 g d'une meringue blanchâtre. Celle-ci est dissoute dans 400 cm3 d'acétate d'éthyle puis traitée par 140 cm3 d'une solution aqueuse d'acide chlorhydrique 0,1N. Le pH de la solution aqueuse est alors ajusté à 4,2 par addition de 400 cm3 de tampon pH 4,2. La phase aqueuse est décantée puis la phase organique est lavée avec 400 cm3 de tampon pH 4,2. Les phases aqueuses sont rassemblées et lavées avec 2 fois 150 cm3 acétate d'éthyle. Après décantation la phase aqueuse est ajustée à pH 7-8 par addition de bicarbonate de sodium puis lavée avec 3 fois 300 cm3 de dichlorométhane. Les phases organiques sont rassemblées puis lavées avec 2 fois 200 cm3 de tampon pH 7,5. La phase aqueuse est lavée avec 50 cm3 de dichlorométhane puis les phases organiques sont rassemblées, séchées sur sulfate de magnésium, filtrées et concentrées à sec sous pression réduite (2,7 kPa) à 30° C pour donner 8,04 g d'un solide jaune clair qui est agité dans 100 cm3 d'éther éthylique, séparé par filtration puis séché sous pression réduite (90 Pa) à 40° C. On obtient ainsi 7,5 g de (diisopropylamino-2 éthyl) sulfinyl-26 pristinamycine $II_B$(isomère $A_2$) sous forme d'une poudre jaune fondant vers 158° C, dont les caractéristiques RMN sont identiques à celles de l'exemple de référence 1.

EXEMPLE DE REFERENCE 3

En opérant d'une manière analogue à celle décrite à l'exemple de référence 1, mais à partir de 6,3 g de (diéthylamino-2 propyl) thio-26 pristinamycine $II_B$, de 0,72 cm3 d'acide trifluoroacétique, de 1,91 g d'acide métachloroperbenzoïque et après purification par chromatographie "flash" [éluant : chloroforme-méthanol (90-10 en volumes)] en recueillant des fractions de 60 cm3 et concentration à sec des fractions 7

à 9 sous pression réduite (2,7 kPa) à 30 °C, on obtient 0,99 g de (diéthylamino-2 propyl) sulfinyl-26 pristinamycine $II_B$ (isomères $A_2$) sous forme d'une poudre jaune fondant vers 150 °C.

Spectre RMN :

1,03 à 1,20 (mt, $-CH_2-CH(C\underline{H}_3)N(CH_2C\underline{H}_3)_2$ et $CH_3$ en 32)

1,76 (s, $-CH_3$ en 33)

3,82 (s, $>CH_2$ en 17)

4,79 (m, $-H_{27}$)

5,53 (d, $-H_{13}$)

6,20 (d, $-H_{11}$)

6,42 (m, $>NH$ en 8)

8,13 (s, $-H_{20}$)

Après concentration à sec des fractions 23 à 35 sous pression réduite (2,7 kPa) à 30 °C, on obtient 0,64 g de (diéthylamino-2 propyl) sulfinyl-26 pristinamycine $II_B$ (isomères $A_1$) sous forme d'une poudre jaune beige fondant vers 160-170 °C.

Spectre RMN :

1,14 (mt, $-N(CH_2C\underline{H}_3)_2$)

1,24 (d large, $C\underline{H}_3-CH-N<$ )

1,73 (s, $-CH_3$ en 33)

3,81 (AB limite, $>CH_2$ en 17)

5,28 (d, $-H_{27}$)

5,43 (d, $-H_{13}$)

6,15 (d, $-H_{11}$)

6,88 (m, $>NH$ en 8)

8,10 (s, $-H_{20}$)

La (diéthylamino-2 propyl) thio-26 pristinamycine $II_B$ peut être préparée de la manière suivante :

En opérant d'une manière analogue à celle décrite à l'exemple 1 de la demande de brevet européen EP 135410, mais à partir de 3,15 g de pristinamycine $II_A$ et 1,8 g de diéthylamino-2 propanethiol et après purification par chromatographie "flash" [éluant : chlorure de méthylène-méthanol (90-10 en volumes )] en recueillant des fractions de 20 cm3 et concentration à sec des fractions 3 à 5 sous pression réduite (2,7 kPa) à 30 °C, on obtient 1,4 g de (diéthylamino-2 propyl) thio-26 pristinamycine $II_B$ sous forme d'une poudre jaune fondant vers 160 °C.

Spectre RMN :

1 (m, 9H : $-H_{32}$ + $-N(CH_2C\underline{H}_3)_2$)

2,50 (m, 6H : $-S-C\underline{H}_2-CH-N(C\underline{H}_2CH_3)_2$)

3,30 (m, 1H : $-H_{26}$)

4,70 (d, 1H : $-H_{27}$)

8,12 (s, 1H : $-H_{20}$)

Le diéthylamino-2 propanethiol peut être préparé de la manière suivante :

A une solution de 29,5 g de dichlorhydrate d'S-isothiouréido-1 diéthylamino-2 propane dans 150 cm3 d'eau distillée, on ajoute 25 cm3 d'une solution aqueuse de soude 10N. Le mélange est porté à 100° C pendant 1 heure, refroidi à 20° C, ajusté à pH 9 par addition de 8 cm3 d'une solution aqueuse d'acide chlorhydrique 12 N, puis extrait par 3 fois 100 cm3 d'éther éthylique. Les phases éthérées sont rassamblées, séchées sur carbonate de potassium, filtrées puis concentrées à sec sous pression réduite (2,7 kPa) à 30° C. Le mélange est purifié par distillation. On obtient 5,8 g de diéthylamino-2 propanethiol-1 sous forme d'un liquide incolore. [Eb (2,7 kPa) = 78° C].

Le dichlorhydrate d'S-isothiouréido-1 diéthylamino-2 propane peut être préparé de la manière suivante :

A une solution de 41 g de chlorhydrate de chlor-1 diéthylamino-2 propane dans 200 cm3 de diméthylformamide, on ajoute 16, 7 g de thiourée. Le mélange est porté à 100° C pendant 30 minutes, puis refroidi à 20° C. Le précipité blanc formé est recueilli par filtration, lavé avec 3 fois 20 cm3 de diméthylformamide puis 3 fois 20 cm3 d'éther éthylique. On obtient 29,6 g de dichlorhydrate d'S-isothiouréido-1 diéthylamino-2 propane sous forme de cristaux blancs fondant à 247-249° C.

Le chlorhydrate de chloro-1 diéthylamino-2 propane peut être obtenu de la manière suivante :

A 100 cm3 de chlorure de thionyle on ajoute en 15 minutes 45,2 g de chlorhydrate de diéthylamino-2 propanol puis on chauffe à 80° C. Après 2 heures d'agitation, le chlorure de thionyle en excès est distillé et le résidu repris par 200 cm3 d'éther éthylique. Le chlorhydrate de chloro-1 diéthylamino-2 propane cristallise. On obtient après filtration 48,2 g de cristaux blancs fondant à 112° C.

Le chlorhydrate de diéthylamino-2 propanol peut être obtenu de la manière suivante :

A une suspension maintenue sous azote de 10,6 g d'hydrure de lithium et d'aluminium dans 1 litre d'éther éthylique on ajoute lentement à 20° C une solution de 65 g de diéthylamino-2 propionate d'éthyle dans 330 cm3 d'éther éthylique. La réaction est maintenue à une température de 35° C pendant 5 heures, puis abaissée à 0° C. On ajoute alors goutte à goutte à 0° C, 12,4 cm3 d'eau, 9,1 cm3 d'une solution aqueuse de soude 5N puis 41,3 cm3 d'eau, on agite 30 minutes puis le mélange est filtré sur verre fritté puis lavé à l'éther éthylique. La phase éthérée est séchée sur carbonate de potassium, filtrée puis concentrée à sec sous pression réduite (2,7 kPa) à 30° C. On obtient 43,8 g d'un liquide jaune qui est dissous dans 200 cm3 d'acétone puis on ajoute 78 cm3 d'une solution 4,5N d'acide chlorhydrique gazeux dans l'éther éthylique. Le chlorhydrate de diéthylamino-2 propanol cristallise. On obtient après filtration, 45,2 g de cristaux blancs fondant à 97-100° C.

Le diéthylamino-2 propionate d'éthyle peut être obtenu selon BRAUN et coll., Beilstein, 61, 1425 (1928).

EXEMPLE DE REFERENCE 4

On opère d'une manière analogue à celle décrite à l'exemple de référence 2 mais à partir de 4 g de (diéthylamino-2 propyl) thio-26 pristinamycine $II_B$ (isomères A), de 1,16 g d'acide métachloroperbenzoïque à 98 % et de 1 g de bicarbonate de sodium solide. Après purification par chromatographie "flash" [éluant : chloroformeméthanol (93-7) en volumes] et concentration à sec des fractions 21 à 48 sous pression réduite (2,7 kPa) à 30° C, en recueillant des fractions de 25 cm3, on obtient 2,69 g de (diéthylamino-2 propyl) sulfinyl-26 pristinamycine $II_B$ (isomères $A_2$) sous forme d'une poudre jaune ayant des caractéristiques identiques à celles du produit obtenu à l'exemple de référence 3.

La (diéthylamino-2 propyl) thio-26 pristinamycine $II_B$ (isomère A) peut être obtenue en opérant d'une manière analogue à celle décrite à l'exemple de référence 1 mais à partir de 15 g de pristinamycine $II_A$ et de 4,62 g de diéthylamino-2 propanethiol. Après purification par chromatographie "flash" [éluant : chloroformeméthanol (90-10 en volumes)] et concentration à sec des fractions 27 à 52 sous pression réduite (2,7

kPa) à 30 °C, en recueillant des fractions de 40 cm3, on obtient 12 g d'un solide jaune qui est agité dans 60 cm3 d'éther éthylique, filtré puis séché. On obtient 8,2 g de (diéthylamino-2 propyl) thio-26 pristinamycine $II_B$ (isomère A) sous forme d'une poudre jaune clair fondant vers 122 °C.

**Spectre RMN :**

1 à 1,15 (mt, $-CH_3$ éthyl + $C\underline{H}_3-CH-N(C_2H_5)_2$)

1,70 (s, $-CH_3$ en 33)

2,35 à 2,60 (mt, $-N\begin{matrix} C\underline{H}_2-CH_3 \\ C\underline{H}_2-CH_3 \end{matrix}$ )

2,50 à 3,10 (mt, $-SCH_2CH<$)

2,75 (mt, $-H_4$)

2,89 et 3,05 (2dd } $>CH_2$ en 15)
2,92 et 3,08 (2dd }

3,30 (mt } $-H_{26}$)
3,37 (mt }

3,80 (s, $>CH_2$ en 17)

4,69 (d } $-H_{27}$)
4,71 (d }

5,45 (d, $-H_{13}$)

6,13 (d } $-H_{11}$)
6,14 (d }

6,4 à 6,60 (mt, $>NH$ en 8)

6,51 (dd } $-H_5$)
6,53 (dd }

8,09 (s, $-H_{20}$)

EXEMPLE DE REFERENCE 5

On opère d'une manière analogue à celle décrite à l'exemple de référence 2 mais à partir de 4,58 g de (diéthylamino-1 propyl-2) thio-26 pristinamycine $II_B$ (isomères A), de 1,29 g d'acide métachloroperbenzoïque a 98 % et de 1,14 g de bicarbonate de sodium solide. Après purification par chromatographie "flash" [éluant : chloroformeméthanol (97-3) en volumes] en recueillant des fractions de 20 cm3 et concentration à sec sous pression réduite (2,7 kPa) à 30 °C respectivement des fractions 59 à 77 et des fractions 79 à 97, on obtient : à partir des fractions 79 à 97, 1,47 g de (diéthylamino-1 propyl-2) sulfinyl-26 pristinamycine $II_B$ - (premier isomère) sous forme d'un solide jaune clair fondant vers 132 °C

Spectre RMN :

1,02 (t, $-CH_3$ éthyl)

1,34 (d, $C\underline{H}_3-\overset{}{C}H-CH_2N(C_2H_5)_2$)

1,72 (s, $-CH_3$ en 33)

2,5 à 2,7 (mt, $-CH_2-N\begin{matrix} CH_2- \\ CH_2- \end{matrix}$ )

2,77 (mt, $-H_4$)

2,87 et 3,09 (2dd, $>CH_2$ en 15)

2,97 (mt, $-\underset{\downarrow \atop O}{S}-CH<$ )

3,72 (mt, $-H_{26}$)

3,80 (s, $>CH$ en 17)

4,92 (mt, $-H_{27}$)

5,43 (d, $-H_{13}$)

6,15 (d, $-H_{11}$)

6,72 (dd, $>NH$ en 8)

8,06 (s, $-H_{20}$)

et à partir des fractions 59 à 77, 1,07 g de (diéthylamino-1 propyl-2) sulfinyl-26 pristinamycine $II_B$ - (deuxième isomère) sous forme d'un solide jaune clair fondant vers 128 °C.

Spectre RMN :

1,72 (s, $CH_3$ en 33)

3,4 (mt, $-H_{26}$)

3,79 (s, $CH_2$ en 17)

4,74 (mt, $-H_{27}$)

5,48 (d, $-H_{13}$)

6,18 (d, $-H_{11}$)

6,80 (mt, $>NH$ en 8)

8,09 (s, $-H_{20}$)

La (diéthylamino-1 propyl-2) thio-26 pristinamycine $II_B$ (isomères A) peut être obtenue en opérant d'une manière analogue à celle décrite à l'exemple 1 mais à partir de 13 g de pristinamycine $II_A$ et de 4 g de diéthylamino-1 propane-2 thiol. Après purification par chromatographie "flash" [éluant : chloroforme-méthanol (90-10 en volumes)] et concentration à sec des fractions 46 à 55 sous pression réduite (2,7 kPa) à 30 °C en recueillant des fractions de 50 cm3, on obtient 8 g d'un solide jaune pâle qui est cristallisé dans 30 cm3 d'acétonitrile. Après filtration et séchage, on obtient 5,91 g de (diéthylamino-1 propyl-2) thio-26 pristinamycine $II_B$ (isomères A) sous forme de cristaux blancs fondant à 136 °C.

Spectre RMN :

0,9 à 1,10 (mt, $-N(CH_2C\underline{H}_3)_2$)

1,33 à 1,37 (2d, $C\underline{H}_3-\underset{|}{C}H-CH_2N<$)

1,7 (s, $-CH_3$ en 33)

2,4 à 2,65 (mt, $-CH_2N\begin{matrix} CH_2- \\ CH_2- \end{matrix}$)

2,76 (mt, $-H_4$)

3 (mt, $-S-CH<$ )

2,9 et 3,1 (2dd, $>CH_2$ en 15)

3,52 (mt, $-H_{26}$)

3,81 (s, $>CH_2$ en 17)

4,78 (mt, $-H_{27}$)

5,46 (d, $-H_{13}$)

6,14 (d, $-H_{11}$)

6,40 (mf, $>$ NH en 8)

8,09 et 8,10 (2s, $-H_{20}$)

Le diéthylamino-1 propane-2 thiol peut être obtenu suivant la méthode décrite par R.T. WRAGG, J. Chem. Soc. (C), 2087 (1969).

EXEMPLE DE REFERENCE 6

On opère d'une manière analogue à celle décrite à l'exemple de référence 2 mais à partir de 1,7 g de [diméthylamino-2 butyl 2(R)] thio-26 pristinamycine $II_B$ (isomère A), de 0,50 g de bicarbonate de sodium, de 0,45 g d'acide méthachloroperbenzoïque à 98 %. Après purification par chromatographie "flash" [éluant : acétate d'éthyleméthanol (85-15 en volumes)] et concentration à sec des fractions 35 à 58 sous pression réduite (2,7 kPa) à 30 °C, on obtient 1,1 g d'un solide blanc qui est agité dans 30 cm3 d'éther éthylique. Après filtration et séchage, on obtient 0,95 g de [diméthylamino-2 butyl 2(R)] sulfinyl-26 pristinamycine $II_B$ - (isomère $A_2$) sous forme d'un solide blanc fondant vers 126 °C.

**Spectre RMN :**

1 (mt, $>N-\underset{|}{C}H-CH_2C\underline{H}_3$)

1,45 à 1,75 (mt, $>N-\underset{|}{C}H-C\underline{H}_2CH_3$)

1,78 (s, $-CH_3$ en 33)

2,50 à 3,05 (mt, $-\underset{\downarrow \atop O}{S}-CH_2-CH<$ et $-H_4$)

2,93 et 3,14 (2dd, $>CH_2$ en 15)

3,31 (mt, $-H_{26}$)

3,84 (s, $>CH_2$ en 17)

4,84 (d, $-H_{27}$)

5,51 (d, $-H_{13}$)

6,19 (d, $-H_{11}$)

6,30 (dd, $>NH$ en 8)

8,15 (s, $-H_{20}$)

La [diméthylamino-2 butyl 2(R)] thio-26 pristinamycine $II_B$ (isomère A) peut être obtenue en opérant d'une manière analogue à celle décrite à l'exemple de référence 1 mais à partir de 8 g de pristinamycine $II_A$ et 2,3 g de diméthylamino-2 butanethiol 2(R). Après purification par chromatographie "flash" [éluant : dichlorométhane-méthanol (90-10 en volumes)] et concentration à sec des fractions 36 à 55 sous pression réduite (2,7 kPa) à 30 °C, on obtient 3 g de [diméthylamino-2 butyl 2(R)] thio-26 pristinamycine $II_B$ (isomère A) sous forme d'un solide jaune clair fondant vers 120 °C.

La cristallisation de 0,9 g de ce produit dans 5 cm3 d'acétonitrile conduit après séparation par filtration à 0,2 g de [diméthylamino-2 butyl 2(R)] thio-26 pristinamycine $II_B$ (isomère A) sous forme de cristaux blancs fondant à 122 °C.

**Spectre RMN :**

1 (mt, $>N-\underset{|}{C}H-CH_2C\underline{H}_3$)

1,4 à 1,7 (mt, $>N-\underset{|}{C}H-C\underline{H}_2CH_3$)

1,72 (s, $-CH_3$ en 33)

2,30 (s, $-N(CH_3)_2$)

2,5 à 2,85 (mt, $-S-CH_2-CH<$ et $-H_4$)

2,93 et 3,10 (2dd, $>CH_2$ en 15)

3,34 (d large, $-H_{26}$)

3,83 (s, $>CH_2$ en 17)

4,76 (s large, $-H_{27}$)

5,48 (d, $-H_{13}$)

6,14 (d, $-H_{11}$)

6,26 (dd, $>NH$ en 8)

8,13 (s, $-H_{20}$)

Le diméthylamino-2 butanethiol (R) peut être obtenu d'une manière analogue à celle décrite ci-après à l'exemple de référence 7, à partir de 52,4 g de triphénylphosphine, de 40 cm3 de diisopropylazodicarboxylate, de 12 g de diméthylamino-2 butanol (R) et de 15,2 cm3 d'acide thiolacétique (dans ce cas le thioester intermédiaire est hydrolysé directement lors de la chromatographie sur gel de silice).

Après purification par chromatographie "flash" [éluant : dichlorométhane : 1000 cm3, puis dichlorméthane-méthanol (85-15 en volumes) : 2000 cm3, puis dichlorométhane-méthanol (80-20 en volumes) : 4000 cm3] en recueillant des fractions de 100 cm3 et concentration à sec sous pression réduite des fractions 42 à 60, on obtient 14 g d'une huile jaune qui est purifiée par distillation. On obtient ainsi 2,4 g de diméthylamino-2 butanethiol (R) sous forme d'un liquide incolore [Eb (4kPa) = 70-75 °C].

Le diméthylamino-2 butanol-1 (R) peut être obtenu d'une manière identique à celle décrite par M. WENGHOEFER et coll., J. Heterocycl. Chem., 7(6), 1407 (1970).

EXEMPLE DE REFERENCE 7

En opérant d'une manière analogue à celle décrite à l'exemple de référence 2 mais à partir de 2,67 g de [diméthylamino-2 phényl-3 propyl (2S)] thio-26 pristinamycine $II_B$ (isomère A), de 0,7 g de bicarbonate de sodium et de 0,7 g d'acide métachloroperbenzoïque à 98 %, après purification par chromatographie "flash" [éluant : chloroforme-méthanol (90-10 en volumes)] en recueillant des fractions de 20 cm3 et concentration à sec des fractions 19 à 23 sous pression réduite (2,7 kPa) à 30 °C, on obtient 1,3 g d'un solide jaune clair qui est agité dans 50 cm3 d'éther éthylique, séparé par filtration pour donner 1,18 g de [diméthylamino-2 phényl-3 propyl (2S)] sulfinyl-26 pristinamycine $II_B$ (isomère $A_2$) sous forme d'un solide jaune clair fondant vers 150 °C.

Spectre RMN (400 MHz, $CDCl_3$)

1,73 (s, $-CH_3$ en 33)

2,4 à 2,6 (mt,

2,8 à 3,15 (mt,

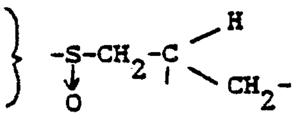

)

2,44 (s, $-N(CH_3)_2$)

2,77 (mt, $-H_4$)

2,89 et 3,1 (2dd, $>CH_2$ en 15)

3,18 (mt, $-H_{26}$)

3,82 (s, $>CH_2$ en 17)

4,68 (d, $-H_{27}$)

5,51 (d, $-H_{13}$)

6,19 (d, $-H_{11}$)

6,50 (dd, $>NH$ en 8)

7,18 (d, -H en ortho du phényle)

7,23 (t, -H en para du phényle)

7,31 (t, -H en méta du phényle)

8,13 (s, $-H_{20}$)

On obtient une solution aqueuse à 1 % de [diméthylamino-2 phényl-3 propyl(2S)] sulfinyl-26 pristinamycine $II_B$ (isomère $A_2$) avec :

- produit ......................... 30 mg
- acide chlorhydrique 0,1N ......... 0,45 cm3
- eau distillée .................qsp 3 cm3

La [diméthylamino-2 phényl-3 propyl (2S)] thio-26 pristinamycine $II_B$ (isomère A) peut être préparée en opérant d'une manière analogue à celle décrite à l'exemple 1 pour la préparation de la matière de départ mais à partir de 7,13 g de pristinamycine $II_A$ et 2,65 g de diméthylamino-2 phényl-3 propanethiol (S) et après purification par chromatographie "flash" [éluant : acétate d'éthyleméthanol (80-20 en volumes)] en recueillant des fractions de 60 cm3 et concentration à sec des fractions 33 à 43 sous pression réduite (2,7 kPa) à 30 °C, on obtient 4,6 g d'un solide jaune clair qui est agité dans 50 cm3 d'éther éthylique, filtré puis séché sous pression réduite (90 Pa) à 45 °C. On obtient ainsi 3,6 g de [diméthylamino-2 phényl-3 propane (2S)] thio-26 pristinamycine $II_B$ (isomère A) sous forme d'une poudre jaune pâle fondant vers 110 °C.

Spectre RMN :

1,69 (s, $-CH_3$ en 33)

2,38 (s, $-N(CH_3)_2$)

2,35 à 3,05 (mt, 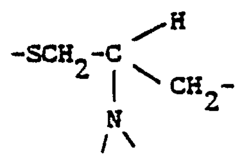 )

2,73 (mt, $-H_4$)

2,89 et 3,10 (2dd, $>CH_2$ en 15)

3,26 (d large, $-H_{26}$)

3,81 (s, $>CH_2$ en 17)

4,68 (s large, $-H_{27}$)

5,47 (d, $-H_{13}$)

6,12 (d, $-H_{11}$)

6,27 (mf, $>NH$ en 8)

7,18 (d, -H en ortho du phényle)

7,21 (t, -H en para du phényle)

7,30 (t, -H en méta du phényle)

8,11 (s, $-H_{20}$)

Le diméthylamino-2 phényl-3 propanethiol (S) peut être préparé de la manière suivante :

A 20 g de diméthylamino-2 phényl-3 propanethiolacétate (S) (brut) en solution dans 50 cm3 de méthanol on ajoute sous atmosphère d'azote, 0,2 g de méthylate de sodium et on chauffe à reflux pendant 2 heures. Le mélange est ensuite concentré à sec sous pression réduite (2,7 kPa) à 30 °C pour donner un liquide qui est purifié par distillation. On obtient 2,4 g de diméthylamino-2 phényl-3 propanethiol (S) sous forme d'un liquide incolore [Eb (14 Pa) = 95 °C] qui est utilisé tel quel dans la réaction suivante.

Le diméthylamino-2 phényl-3 propanethiolacétate (S) peut être préparé de la manière suivante :

On ajoute à 0 °C, sous atmosphère d'azote, 41,97 g de triphénylphosphine et 310 cm3 de tétrahydrofuranne puis on additionne goutte à goutte 31,5 cm3 de diisopropylazodicarboxylate et laisse agiter une demi heure à 0 °C. A la suspension blanche obtenue on ajoute goutte à goutte un mélange de 15 g de diméthylamino-2 phényl-3 propanol (S) et de 11,44 cm3 d'acide thiolacétique en solution dans 160 cm3 de tétrahydrofuranne. Après 1 heure d'agitation à 0 °C puis 1 heure 30 minutes à 25 °C, le mélange est concentré à sec sous pression réduite (2,7 kPa) à 30 °C. L'huile obtenue est additionnée de 190 cm3 de

méthanol, le solide blanc qui précipite est éliminé par filtration et le filtrat est concentré à sec sous pression réduite (2,7 kPa) à 30 °C. Le résidu est alors agité avec 200 cm3 d'oxyde d'isopropyle, le solide blanc précipité est à nouveau éliminé par filtration et le filtrat est concentré pour donner 45 g d'une huile jaune qui est purifiée par chromatographie "flash" [éluant : dichlorométhane-méthanol (90-10 en volumes)] en recueillant des fractions de 100 cm3. Après concentration à sec des fractions 37 à 55 sous pression réduite (2,7 kPa) à 30 °C, on obtient 10,4 g de diméthylamino-2 phényl-3 propanethiolacétate (S) sous forme d'une huile jaune orangée (contenant de l'oxyde de triphénylphosphine).

Le diméthylamino-2 phényl-3 propanol (S) peut être préparé par analogie avec la méthode décrite par T. HAYASHI et coll., J. Org. Chem., 48, 2195 (1983).

EXEMPLES DE REFERENCE 8 A 26

On opérant de manière analogue à celle de l'exemple de référence 2, on prépare les produits suivants :

| Exemple de référence | R | n | 1) Isomère<br>2) Point de fusion |
|---|---|---|---|
| 8 | $-(CH_2)_2\ N(C_2H_5)_2$ | 1 | 1) isomère $A_2$<br>2) F ∿ 172°C |
| 9 | $-(CH_2)_2\ N(CH_3)_2$ | 1 | 1) isomère $A_2$ 70 %<br>isomère $A_1$ 15 %<br>isomère $B_1$ 7 %<br>isomère $B_2$ 8 %<br>2) F ∿ 150°C |
| 10 | $-(CH_2)\ N-C_2H_5$<br>\|<br>$CH_3$ | 1 | 1) isomère $A_2$<br>2) F ∿ 145°C |
| 11 | $-(CH_2)_3\ N(CH_3)_2$ | 1 | 1) isomère $A_2$ 45 %<br>isomère $B_2$ 35 %<br>isomère $B_1$ 15 %<br>2) F ∿ 165°C |
| 12 | $-(CH_2)_2-$ 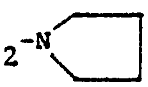 | 1 | 1) isomère $A_1$ 60 %<br>isomère $A_2$ 25 %<br>isomère $B_1$ 15 %<br>2) F ∿ 175°C |
| 13 | $-(CH_2)_2-$ 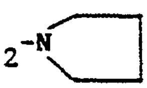 | 1 | 1) isomère $A_2$ 75 %<br>isomère $A_1$ 5 %<br>isomère $B_1$ 10 %<br>isomère $B_2$ 10 %<br>2) F ∿ 145°C |
| 14 | $-(CH_2)_2-$ 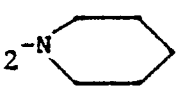 | 1 | 1) isomère $A_2$ 90 %<br>isomère $A_1$ 10 %<br>2) F ∿ 162°C |
| 15 | $-(CH_2)_2-$ 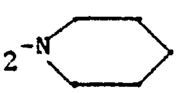 | 1 | 1) isomère $A_1$ 50 %<br>isomère $A_2$ 50 %<br>2) F ∿ 152°C |
| 16 | $-(CH_2)_2-$ 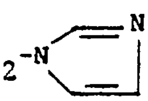 | 1 | 1) isomère $A_2$<br>2) F ∿ 170°C |

| Exemple de référence | R | n | 1) Isomère<br>2) Point de fusion |
|---|---|---|---|
| 17 | $-(CH_2)_2-$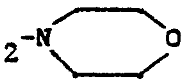 | 1 | 1) isomère $A_2$<br>2) F $\sim$ 126°C |
| 18 | $-(CH_2)_2$ NH $C_4H_9$ | 1 | 1) isomère $A_2$ 70 %<br>isomère $B_1$ 15 %<br>isomère $B_2$ 15 %<br>2) F $\sim$ 140°C |
| 19 | $-(CH_2)_2$ NH $C_4H_9$ | 1 | 1) isomère $A_1$ 85 %<br>isomère $B_1$ 15 %<br>2) F $\sim$ 170°C |
| 20 | $-(CH_2)_2$ NH $C_4H_9$ | 1 | 1) isomère $B_1$ 65 %<br>isomère $B_2$ 35 %<br>2) F $\sim$ 140°C |
| 21 | $-(CH_2)_2$ NH $C_{10}H_{21}$ | 1 | 1) isomère $A_2$ 80 %<br>2) F $\sim$ 128°C |
| 22 | $-(CH_2)_2$ NH $C_{10}H_{21}$ | 1 | 1) isomère $A_2$ 50 %<br>isomère $A_1$ 15 %<br>isomère $B_1$ 20 %<br>isomère $B_2$ 15 %<br>2) F $\sim$ 124°C |
| 23 | $-(CH_2)_2-NH-$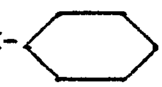 | 1 | 1) isomère $A_2$ 90 %<br>2) F $\sim$ 166°C |
| 24 | $-(CH_2)_2-$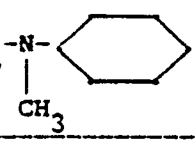 | 1 | 1) isomère $A_2$<br>2) F $\sim$ 126°C |
| 25 | $-(CH_2)_2OCO$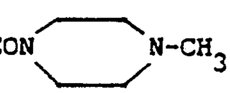 | 1 | 1) isomère $A_2$<br>2) F $\sim$ 135°C |
| 26 | $-(CH_2)_2OCO$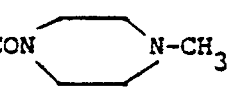 | 1 | 1) isomère $A_1$<br>2) F $\sim$ 140°C |

EXEMPLE DE REFERENCE 27

En opérant d'une manière analogue à celle décrite à l'exemple de référence 1, mais à partir de 7,8 g de [méthyl-1 pyrrolidinyl-2(S)] méthylthio-26 pristinamycine $II_B$ (isomère A), de 0,91 cm3 d'acide trifluoracétique, de 2,4 g d'acide métachloroperbenzoïque et après purification par chromatographie "flash" [éluant : chloroformeméthanol (90-10 en volumes)] en recueillant des fractions de 60 cm3 et concentration à sec des fractions 26 à 36 sous pression réduite (2,7 kPa) à 30 ° C, on obtient 2,3 g de [méthyl-1 pyrrolidinyl-2(S)] méthylsulfinyl-26 pristinamycine $II_B$ (isomère $A_2$) sous forme d'une poudre jaune clair fondant vers 140 ° C.

**Spectre RMN :**

1,76 (s, $-CH_3$ en 33)

2,48 (s, $>NCH_3$)

1,70 à 2,60 (mt, $-H_{29}$ et $>CH_2$ en 25 et 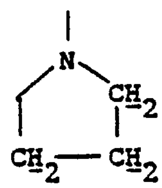 )

2,75 à 3,25 (mt $-S(\rightarrow O)-CH_2-CH<$ )

3,82 (s, $>CH_2$ en 17)

4,81 (d, $-H_{27}$)

5,52 (d, $-H_{13}$)

6,20 (d, $-H_{11}$)

6,42 (dd, $>NH$ en 8)

8,14 (s, $-H_{20}$)

Après concentration à sec des fractions 46 à 59 sous pression réduite (2,7 kPa) à 30 ° C, on obtient 1,1 g de [méthyl-1 pyrrolidinyl-2(S)] méthylsulfinyl-26 pristinamycine $II_B$ (isomère $A_1$) sous forme d'une poudre jaune clair fondant vers 148 ° C.

**Spectre RMN :**

1,73 (s, $-CH_3$ en 33)

1,70 à 2,50 (mt, 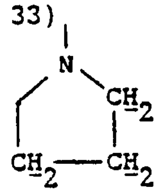 , $-H_{29}$)

2,41 (s, $>N-CH_3$)

2,65 à 3,25 (mt, $>CH_2$ en 15, -H en 4, $-S(\rightarrow O)-CH_2-CH<$ )

3,82 (AB limite, $>CH_2$ en 17)

5,45 (d, $-H_{13}$)

6,17 (d, $-H_{11}$)

8,11 (s, $-H_{20}$)

La (méthyl-1 pyrrolidinyl-2) méthylthio-26 pristinamycine $II_B$ peut être préparée de la manière suivante :

En opérant d'une manière analogue à celle décrite à l'exemple 1 de la demande de brevet européen EP 135410, mais à partir de 10,5 de pristinamycine $II_A$ et 3,14 g de [méthyl-1 pyrrolidinyl-2 (S)] méthanethiol et après purification par chromatographie "flash" [éluant : chloroforme- méthanol (90-10 en volumes)] et concentration à sec des fractions 20 à 35 sous pression réduite (2,7 kPa) à 30° C, on obtient 7,8 g de l'isomère A sous forme d'une poudre jaune fondant à environ 120° C.

**Spectre RMN :**

1,70 (s, $-CH_3$ en 33)

2,38 (s, $>N-CH_3$)

1,70 à 2,50 (mt, $-H_{29}$, $CH_2$ en 25 et 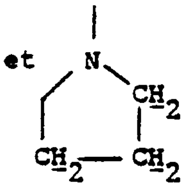 )

2,6 à 3,20 (mt, $-S-CH_2-CH<$)

3,82 (s, $>CH_2$ en 17)

4,73 (d, $-H_{27}$)

5,45 (d, $-H_{13}$)

6,15 (d, $-H_{11}$)

6,41 (dd, $>NH$ en 8)

8,11 (s, $-H_{20}$)

A 25 g de dichlorhydrate de S-[méthyl-1 pyrrolidinyl-2(S) méthyl] isothiouronium brut en solution dans 100 cm3 d'eau distillée, on ajoute 100 cm3 d'une solution aqueuse de soude 4N puis le mélange est agité à 90° C sous atmosphère d'azote pendant 2 heures. Le mélange réactionnel est refroidi à 0° C, additionné de 25 cm3 d'une solution aqueuse d'acide chlorhydrique 12N puis extrait avec 2 fois 200 cm3 de chlorure de méthylène. La phase organique est séchée sur sulfate de sodium, filtrée puis concentrée à sec sous pression réduite (2,7 kPa) à 30° C. On obtient ainsi 5,9 g de [méthyl-1 pyrrolidinyl-2(S)] méthanethiol sous forme d'une huile jaune clair mise en oeuvre dans la suite de la réaction sans purification supplémentaire.

Rf = 0,15 ; chromatoplaque de gel de silice ; éluant : chloroforme-méthanol (90-10 en volumes).

A 11,9 g de chlorhydrate de [méthyl-1 pyrrolidinyl-2(S)] chlorométhane en solution dans 50 cm3 d'éthanol, on ajoute 10,7 g de thiourée puis on agite à reflux pendant 48 heures. Le mélange est concentré à sec sous pression réduite (2,7 kPa) à 40° C. Le résidu est repris avec 100 cm3 d'éthanol chaud puis filtré sur charbon végétal activé. Après concentration à sec du filtrat sous pression réduite (2,7 kPa) à 40° C, on obtient 25 g d'une huile jaune clair composée du dichlorhydrate de S-[méthyl-1 pyrrolidinyl-2(S) méthyl] isothiouronium et de l'excès de thiourée.

Rf = 0,1 ; chromatoplaque de gel de silice ; éluant : chloroforme-méthanol (90-10 en volumes).

Le chlorhydrate de [méthyl-1 pyrrolidinyl-2(S)] chlorométhane peut être préparé selon la méthode décrite par T. HAYASHI et coll., J. Org. Chem., 48, 2195 (1983).

EXEMPLE DE REFERENCE 28

En opérant d'une manière analogue à celle décrite à l'exemple de référence 1, mais à partir de 2,6 g de (méthyl-1 pipéridinyl-4) thio-26 pristinamycine $II_B$, de 0,3 cm3 d'acide trifluoroacétique, de 0,8 g d'acide métachloroperbenzoïque et après purification par chromatographie "flash" [éluant : chloroforme-méthanol (90-10 en volumes)] en recueillant des fractions de 40 cm3 et concentration à sec des fractions 20 à 35 sous pression réduite (2,7 kPa) à 30° C, on obtient 0,33 g de (méthyl-1 pipéridinyl-4) sulfinyl-26 pristinamycine $II_B$ (isomère $A_2$) sous forme d'une poudre jaune fondant vers 170° C.

**Spectre RMN :**

1,76 (s, $-CH_3$ en 33)

2,2 à 3,00 (mt, 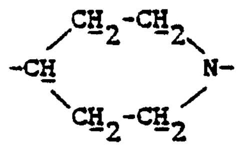 )

2,32 (s, $>N-CH_3$)

3,82 (s, $CH_2$ en 17)

4,85 (d, $-H_{27}$)

5,50 (d, $-H_{13}$)

6,19 (d, $-H_{11}$)

6,37 (dd, >NH en 8)

8,15 (s, $-H_{20}$)

La (méthyl-1 pipéridinyl-4) thio-26 pristinamycine $II_B$ peut être obtenue de la manière suivante :

En opérant d'une manière analogue à celle décrite à l'exemple 1 de la demande de brevet européen EP 135410, mais à partir de 3,15 g de pristinamycine $II_A$ et 1,6 g de méthyl-1 pipéridinethiol-4 et addition de 0,6 g de triéthylamine au mélange réactionnel et après purification par chromatographie "flash" [éluant : chlorure de méthylène-méthanol (92-8 en volumes)] et concentration à sec des fractions 4 à 20 sous pression réduite (2,7 kPa) à 30° C, on obtient 0,9 g de (méthyl-1 pipéridinyl-4) thio-26 pristinamycine $II_B$ sous forme d'une poudre jaune fondant vers 180° C.

**Spectre RMN :**

2,10 (m, 4H : 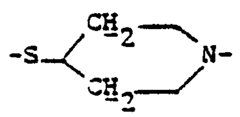 )

2,25 (s, 3H : 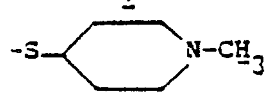 )

2,80 (m, 4H : 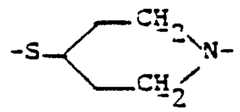 )

3,55 (m, 1H : $-H_{26}$)

4,62 (m, 1H : $-H_{27}$)

7,70 (m, 1H : $-H_8$)

8,10 (s, 1H : $-H_{20}$)

Le méthyl-1 pipéridinethiol-4 peut être préparé par la méthode décrite par H. BARRER et R.E. LYLE, J. Org. Chem., 27, 641 (1962).

EXEMPLE DE REFERENCE 29

A 7,8 g de (diéthylamino-2 éthyl) thio-26 pristinamycine $II_B$ en solution dans 60 cm3 de méthanol, on ajoute à 0° C sous atmosphère d'azote, 0,92 cm3 d'acide trifluoroacétique. Après 15 minutes à 0° C, la

température est élevée à 15 ° C, puis 1,37 g de dioxyde de sélénium est ajouté. Lorsque tout le dioxyde de sélénium est dissous, on ajoute lentement à une température inférieure à 25 ° C, 7 cm3 d'une solution aqueuse d'eau oxygénée à 30 %. Après 1 heure d'agitation à 25 ° C, le mélange réactionnel est refroidi à 10 ° C, additionné de 50 cm3 d'une solution aqueuse saturée de bicarbonate de sodium puis extrait avec 4 fois 50 cm3 de chlorure de méthylène. Les phases organiques sont réunies, séchées sur du sulfate de magnésium, filtrées puis concentrées à sec sous pression réduite (2,7 kPa) à 30 ° C. Le solide jaune obtenu est purifié par chromatographie "flash" [éluant : chloroforme-méthanol (90-10 en volumes)] en recueillant des fractions de 40 cm3. Après concentration à sec sous pression réduite (2,7 kPa) à 30 ° C des fractions 31 à 38, on obtient un solide jaune qui est purifié par chromatographie "flash" [éluant : acétate d'éthyle-méthanol (80-20 en volumes)] en recueillant des fractions de 40 cm3. Après concentration à sec sous pression réduite des fractions 27 à 33, on obtient un solide blanc qui est agité dans 50 cm3 d'éther ethylique, séparé par filtration puis séché sous pression réduite (90 Pa) à 30 ° C. On obtient ainsi 0,5 g de (diethylamino-2 éthyl) sulfonyl-26 pristinamycine $II_B$ (isomère A) sous forme d'un solide blanc fondant vers 150 ° C.

Spectre RMN :

0,97 (d, $-CH_3$ en 30 et 31 et $-CH_3$ de l'ethyle)

1,75 (s, $-CH_3$ en 33)

2,62 (q, 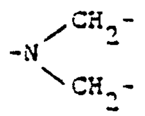 )

3,00 à 3,40 (mt, $-SO_2CH_2CH_2N<$)

3,82 (s, $>CH_2$ en 17)

5,34 (d, $-H_{13}$)

5,43 (d, $-H_{13}$)

6,16 (d, $-H_{11}$)

6,54 (dd, $>NH$ en 8)

8,10 (s, $-H_{20}$)

EXEMPLE DE REFERENCE 30

On opère d'une manière analogue à celle décrite à l'exemple de référence 29, mais à partir de 6,86 g de (diisopropylamino-2 éthyl) thio-26 pristinamycine $II_B$ (isomère A), de 0,77 cm3 d'acide trifluoroacétique, de 1,15 g de dioxyde de sélénium, de 6,33 cm3 d'une solutiion aqueuse d'eau oxygénée à 30 %. Après purification par chromatographie "flash" [éluant : acétate d'éthyle-méthanol (80-20 en volumes)] en recueillant des fractions de 40 cm3 et concentration à sec des fractions 28 à 31 sous pression réduite (2,7 kPa) à 30 ° C, on obtient 0,7 g d'un solide jaune qui est à nouveau purifié par chromatographie "flash" [éluant : acétate d'éthyle-méthanol (85-15 en volumes)] en recueillant des fractions de 30 cm3. Après concentration à sec sous pression réduite des fractions 26 à 33, on obtient un solide jaune qui est agité dans 30 cm3 d'éther éthylique, séparé par filtration puis séché sous pression réduite (90 Pa) à 30 ° C. On obtient 0,6 g de (diisopropylamino-2 éthyl) sulfonyl-26 pristinamycine $II_B$ (isomère A) sous forme d'un solide jaune clair fondant vers 140 ° C.

Spectre RMN :

1,06 (d, $-CH_3$ isopropyle)

1,75 (s, $-CH_3$ en 33)

2,79 (mt, $-H_4$)

2,92 et 3,10 (2dd, $>CH_2$ en 15)

2,7 à 3,30 (mt, $-S(\rightarrow O)_2-CH_2CH_2N(CH<)_2$)

3,52 (d large, $-H_{26}$)

3,82 (s, $>CH_2$ en 17)

5,27 (d fin, $-H_{27}$)

5,47 (d, $-H_{13}$)

6,17 (d, $-H_{11}$)

6,42 (mt, $>NH$ en 8)

8,12 (s, $-H_{20}$)

La présente invention concerne également les médicaments constitués par un produit de formule générale (I), sous forme libre ou sous forme de sel d'addition avec un acide pharmaceutiquement acceptable ; à l'état pur ou sous forme d'une association avec la pristinamycine $II_A$ ou de préférence avec un dérivé soluble de pristinamycine $II_B$ de formule générale (VII), la composition pouvant en outre contenir tout autre produit pharmaceutiquement compatible, pouvant être inerte ou physiologiquement actif. Les médicaments selon l'invention peuvent être utilisés par voie parentérale, orale, rectale ou topique.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Comme compositions solides pour administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration topique peuvent être par exemple des crêmes, pommades,

lotions, collyres, collutoires, gouttes nasales ou aérosols.

En thérapeutique humaine, les produits selon l'invention sont particulièrement utiles dans le traitement des infections d'origine bactérienne. Les doses dépendant de l'effet recherché et de la durée du traitement. Pour un adulte, elles sont généralement comprises entre 2000 et 4000 mg par jour par voie parentérale, particulièrement par voie intraveineuse en perfusion lente.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent des compositions selon l'invention.

EXEMPLE A

On prépare, selon la technique habituelle, une solution injectable pour perfusion contenant 5 g/l de produit actif ayant la composition suivante :

- [quinuclidinyl-3(S)] thiométhyl-5δ pristinamycine $I_A$ ...... 5 g
- solution aqueuse d'acide chlorhydrique 0,1N ............... 49 cm3
- eau distillée ..................................... qsp 1000 cm3

EXEMPLE B

On prépare, selon la technique habituelle, une solution injectable pour perfusion contenant 1 g/l de mélange actif ayant la composition suivante :

- [quinuclidinyl-3(S)] thiométhyl-5δ pristinamycine $I_A$ ........................................ 0,4 g
- (diéthylamino-2 éthyl) sulfonyl-26 pristinamycine $II_B$, isomère A .............................................. 0,6 g
- solution aqueuse d'acide chlorhydrique 0,1N .............. 12,6 cm3
- eau distillée ..................................... qsp 1000 cm3

EXEMPLE C

On prépare, selon la technique habituelle, une solution injectable pour perfusion contenant 1 g/l de mélange actif ayant la composition suivante :

- [quinuclidinyl-3(R)] thiométhyl-5δ pristinamycine $I_A$ .... 0,4 g
- (diisopropylamino-2 éthyl) sulfonyl-26 pristinamycine $II_B$, isomère A ........................................ 0,6 g
- solution aqueuse d'acide chlorhydrique 0,1N .............. 12,3 cm3
- eau distillée ..................................... qsp 1000 cm3

EXEMPLE D

On prépare, selon la technique habituelle, une solution injectable pour perfusion contenant 1 g/l de mélange actif ayant la composition suivante :

- (quinuclidinyl-3) thiométhyl-5δ pristinamycine $I_A$ ....... 0,4 g
- (diéthylamino-2 propyl) sulfinyl-26 pristinamycine $II_B$, isomère A ........................................... 0,6 g
- solution aqueuse d'acide chlorhydrique 0,1N .............. 12,7 cm3
- eau distillée ...................................... qsp 1000 cm3

## Revendications

1. Nouveau dérivé de synergistine caractérisé en ce qu'il répond à la formule générale :

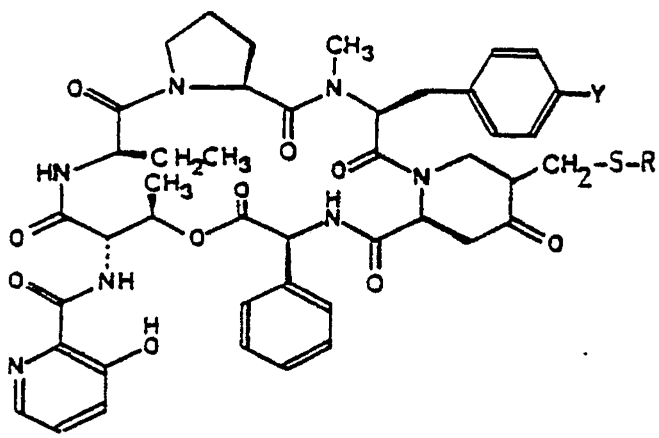

(I)

dans laquelle Y représente un atome d'hydrogène ou un radical diméthylamino et R représente le radical quinuclidinyle-3 ou -4, sous ses formes isomères et leurs mélanges, ainsi que ses sels pharmaceutiquement acceptables.

2. La [quinuclidinyl-3(S)] thiométhyl-5δ pristinamycine $I_A$ ainsi que ses sels pharmaceutiquement acceptables.

3. La [quinuclidinyl-3(R)] thiométhyl-5δ pristinamycine $I_A$ ainsi que ses sels pharmaceutiquement acceptables.

4. La [quinuclidinyl-3(S)] thiométhyl-5δ virginiamycine S ainsi que ses sels pharmaceutiquement acceptables.

5. La [quinuclidinyl-3(R)] thiométhyl-5δ virginiamycine S ainsi que ses sels pharmaceutiquement acceptables.

6. Procédé de préparation d'un produit selon la revendication 1 caractérisé en ce que l'on fait agir un thiol de formule générale :

R-SH (II)

dans laquelle R est défini comme dans la revendication 1 sur un produit de formule générale :

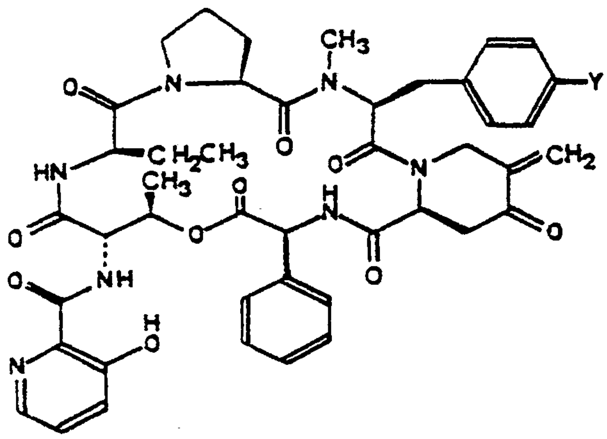

(III)

dans laquelle Y est défini comme à la revendication 1, puis isole le produit, sépare éventuellement ses isomères et le transforme éventuellement en un sel pharmaceutiquement acceptable.

**7.** Composition pharmaceutique caractérisée en ce qu'elle contient au moins un dérivé selon la revendication 1, à l'état pur ou en association avec un ou plusieurs diluants ou adjuvants compatibles et pharmaceutiquement acceptables, et/ou en association avec la pristinamycine $II_A$ ou un dérivé soluble de la pristinamycine $II_B$ de formule générale :

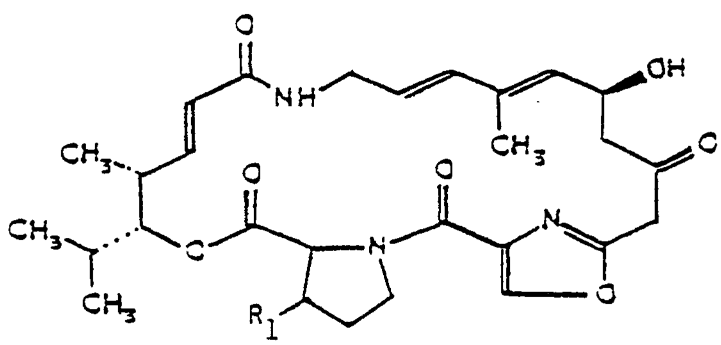

(VII)

dans laquelle $R_1$ représente

1° un radical alcoylthio contenant 1 à 5 atomes de carbone substitué :

- i) par un ou deux radicaux alcoylamino ou dialcoylamino dont les parties alcoyle qui contiennent 1 à 5 atomes de carbone peuvent éventuellement former ensemble avec l'atome d'azote auquel elles sont liées un hétérocycle saturé choisi parmi pyrrolidinyle-1, pipéridino, azétidinyle-1, azépinyle-1, morpholino, thiomorpholino et pipérazinyle-1 (éventuellement substitué par un radical alcoyle contenant 1 à 5 atomes de carbone), ou bien
- ii) par un radical pyrrolidinyle-2 ou 3, pipéridyle-2, 3 ou 4, azétidinyle-2 ou 3 ou azépinyle-2, 3 ou 4,

2° un radical de formule générale :

Het-S-

dans laquelle Het représente un radical pyrrolidinyle-3, pipéridyle-3 ou 4, azétidinyle-3 ou azépinyle-3 ou 4 éventuellement substitué par un radical alcoyle contenant 1 à 5 atomes de carbone
3° un radical dialcoylamino dont les parties alcoyle peuvent éventuellement former ensemble avec l'atome d'azote auquel elles sont liées un hétérocycle saturé choisi parmi pyrrolidinyle-1, pipéridino, azétidinyle-1, azépinyle-1, morpholino, thiomorpholino et pipérazinyle-1 (éventuellement substitué par un radical alcoyle contenant 1 à 5 atomes de carbone),
4° un radical de formule générale :

$$R_2 - \underset{(O)_n}{S} -$$

dans laquelle $R_2$ représente

- soit un radical hétérocyclyle azoté de 4 à 7 chaînons, contenant éventuellement 1 ou plusieurs autres hétéroatomes choisis parmi l'azote, l'oxygène ou le soufre à l'état de sulfoxyde ou de sulfone, et éventuellement substitué par un radical alcoyle,
- soit une chaîne alcoyle contenant 2 à 4 atomes de carbone substituée par 1 ou 2 radicaux choisis parmi phényle, cycloalcoylamino ou N-alcoyl N-cycloalcoyl-amino contenant 3 à 6 chaînons, alcoylamino, dialcoylamino ou dialcoylcarbamoyloxy (les parties alcoyle de ces 2 derniers radicaux pouvant éventuellement former avec l'atome d'azote auquel elles sont rattachées un hétérocycle saturé ou insaturé de 4 à 7 chaînons contenant éventuellement un autre hétéroatome choisi parmi l'azote, l'oxygène ou le soufre à l'état de sulfoxyde ou de sulfone, et éventuellement substitué par un radical alcoyle), ou substituée par un ou plusieurs hétérocycles azotés de 4 à 7 chaînons contenant éventuellement 1 ou 2 autres hétéroatomes choisis parmi l'azote, l'oxygène ou le soufre à l'état de sulfoxyde ou de sulfone, et éventuellement substitués par un radical alcoyle, lesdits hétérocycles étant rattachés à la chaîne qui les porte par l'intermédiaire d'un atome de carbone, étant entendu que l'un au moins de substituants portés par la chaîne alcoyle ci-dessus est un substituant azoté capable de former des sels, soit un radical [méthyl-1 pyrrolidinyl-2 (S)] méthyle, le symbole n est égal à 1 ou 2, les radicaux alcoyle cités ci-dessus étant droits ou ramifiés et contenant, sauf mention spéciale, 1 à 10 atomes de carbone, le cas échéant sous forme de l'un de ses isomères ou leurs mélanges et éventuellement sous forme de sel d'addition avec un acide.

Revendication pour les Etats contractants suivants : AT, ES, GR

**1.** Procédé de préparation d'un nouveau dérivé de synergistine de formule générale :

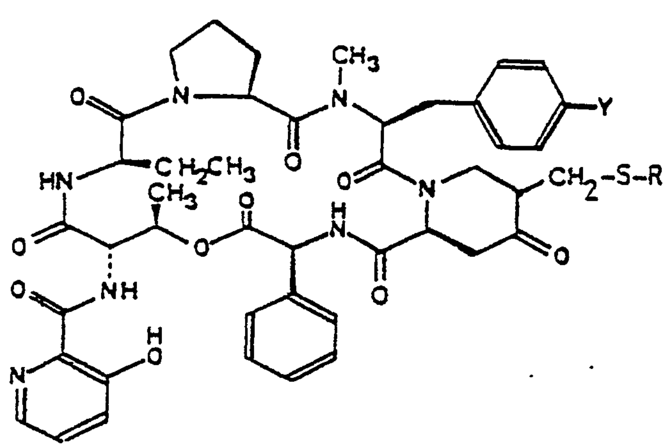

(I)

dans laquelle Y représente un atome d'hydrogène ou un radical diméthylamino et R représente le radical quinuclidinyle-3 ou -4, caractérisé en ce que l'on fait agir un thiol de formule générale :

R-SH

dans laquelle R est défini comme précédemment sur un produit de formule générale :

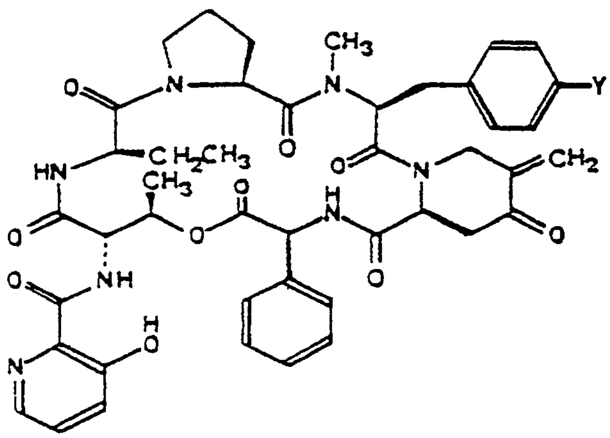

dans laquelle Y est défini comme ci-dessus, puis isole le produit, sépare éventuellement ses isomères et le transforme éventuellement en un sel pharmaceutiquement acceptable.

## Claims

1. New synergistine derivative characterized in that it corresponds to the general formula:

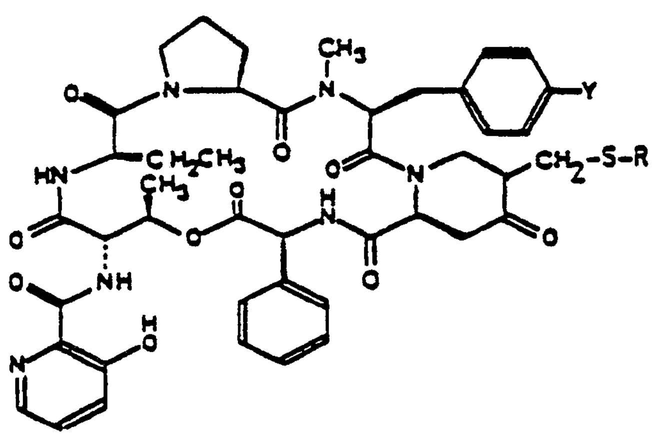

(I)

in which Y denotes a hydrogen atom or a dimethylamino radical and R denotes the 3- or 4-quinuclidinyl radical, in its isomeric forms and mixtures thereof, and its pharmaceutically acceptable salts.

2. [(S)3-Quinuclidinyl]-5δ-thiomethylpristinamycin $I_A$ and its pharmaceutically acceptable salts.

3. [(R)3-Quinuclidinyl]-5δ-thiomethylpristinamycin $I_A$ and its pharmaceutically acceptable salts.

4. [(S)3-Quinuclidinyl]-5δ-thiomethylvirginiamycin S and its pharmaceutically acceptable salts.

5. [(R)3-Quinuclidinyl]-5δ-thiomethylvirginiamycin S and its pharmaceutically acceptable salts.

6. Process for the preparation of a product according to Claim 1, characterized in that a thiol of general formula:

**R-SH** **(II)**

in which R is defined as in Claim 1 is reacted with a product of general formula:

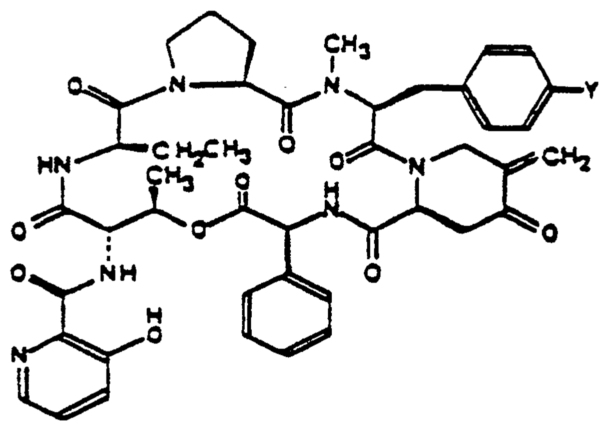

(III)

in which Y is defined as in Claim 1, and the product is then isolated, its isomers are optionally separated and it is optionally converted into a pharmaceutically acceptable salt.

7. Pharmaceutical composition characterized in that it contains at least one derivative according to Claim 1, in the pure state or in combination with one or more compatible and pharmaceutically acceptable diluents or adjuvants, and/or in combination with pristinamycin $II_A$ or a soluble derivative of pristinamycin $II_B$ of general formula:

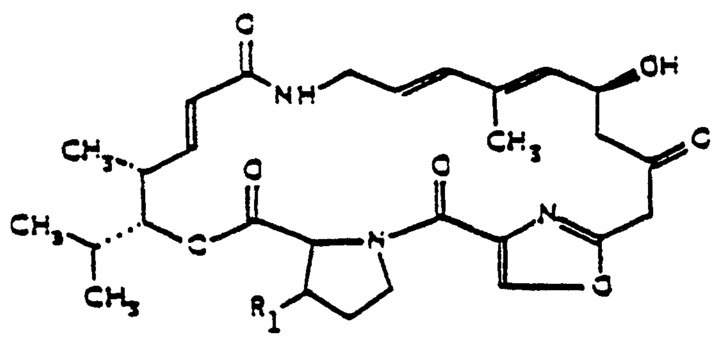

(VII)

in which $R_1$ denotes

1) an alkylthio radical containing 1 to 5 carbon atoms substituted:

- i) by one or two alkylamino or dialkylamino radicals in which the alkyl parts which contain 1 to 5 carbon atoms may optionally form together with the nitrogen atom to which they are bonded a saturated heterocyclic ring chosen from 1-pyrrolidinyl, piperidino, 1-azetidinyl, 1-azepinyl, morpholino, thiomorpholino and 1-piperazinyl (optionally substituted by an alkyl radical containing 1 to 5 carbon atoms), or else
- ii) by a 2- or 3-pyrrolidinyl, 2-, 3- or 4-piperidyl, 2- or 3-azetidinyl or 2-, 3- or 4-azepinyl radical,

2) a radical of general formula:

Het-S-

in which Het denotes a 3-pyrrolidinyl, 3- or 4-piperidyl, 3-azetidinyl or 3- or 4-azepinyl radical optionally substituted by an alkyl radical containing 1 to 5 carbon atoms,

3) a dialkylamino radical in which the alkyl parts may optionally form together with the nitrogen atom to which they are bonded a saturated heterocyclic ring chosen from 1-pyrrolidinyl, piperidino, 1-azetidinyl, 1-azepinyl, morpholino, thiomorpholino and 1-piperazinyl (optionally substituted by an alkyl radical containing 1 to 5 carbon atoms),

4) a radical of general formula:

$$R_2 - \underset{(O)_n}{S} -$$

in which $R_2$ denotes

- either a 4- to 7-membered nitrogenous heterocyclic ring radical optionally containing 1 or more other heteroatoms chosen from nitrogen, oxygen or sulphur in the form of sulphoxide or of sulphone, and optionally substituted by an alkyl radical,
- or an alkyl chain containing 2 to 4 carbon atoms, substituted by 1 or 2 radicals chosen from phenyl, cycloalkylamino or N-alkyl-N-cycloalkylamino containing 3 to 6 chain members, alkylamino, dialkylamino or dialkylcarbamoyloxy (it being possible optionally for the alkyl parts of these 2 last radicals to form with the nitrogen atom to which they are attached a 4- to 7-membered, saturated or unsaturated heterocyclic ring optionally containing another heteroatom chosen from nitrogen, oxygen or sulphur in the form of sulphoxide or of sulphone, and optionally substituted by an alkyl radical), or substituted by one or more 4- to 7-membered nitrogenous heterocyclic rings optionally containing 1 or 2 other heteroatoms chosen from nitrogen, oxygen or sulphur in the form of sulphoxide or of sulphone, and optionally substituted by an alkyl radical, the said heterocyclic rings being attached to the chain which carries them through the intermediary of a carbon atom, it being understood that at least one of the substituents carried by the above alkyl chain is a nitrogenous substituent capable of forming salts, or a [(S)1-methyl-2-pyrrolidinyl]methyl radical, the symbol n is equal to 1 or 2, the alkyl radicals referred to above being straight or branched and, unless especially mentioned, containing 1 to 10 carbon atoms, if appropriate in the form of one of its isomers or mixtures thereof and optionally in the form of an addition salt with an acid.

Claim for the following Contracting States : AT, ES, GR

**1.** Process for the preparation of a new synergistine derivative of general formula:

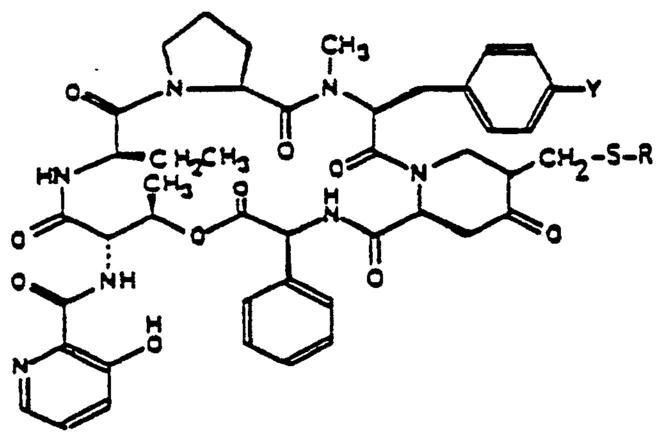

(I)

in which Y denotes a hydrogen atom or a dimethylamino radical and R denotes the 3- or 4-quinuclidinyl radical, characterized in that a thiol of general formula:

R-SH

in which R is defined as above is reacted with a product of general formula:

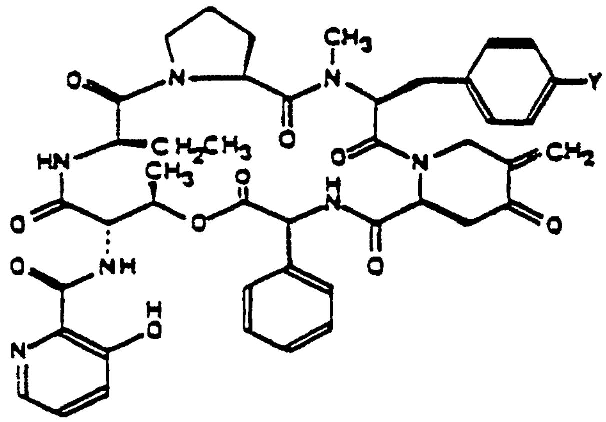

in which Y is defined as above, and the product is then isolated, its isomers are optionally separated, and it is optionally converted into a pharmaceutically acceptable salt.

## Ansprüche

1. Neues Synergistin-Derivat, dadurch gekennzeichnet, daß es der allgemeinen Formel (I)

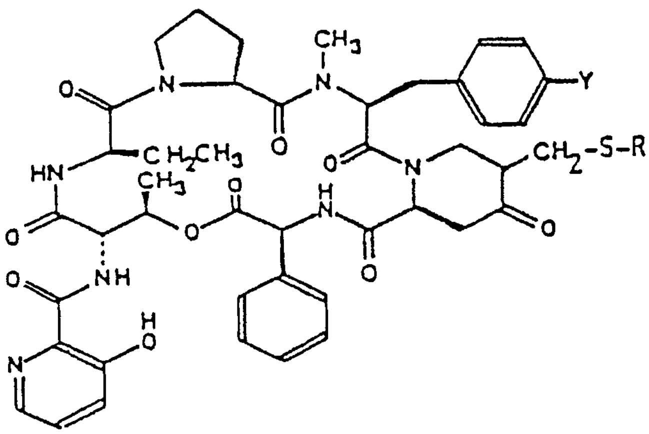

(I)

entspricht, worin Y ein Wasserstoffatom oder einen Dimethylaminorest bedeutet und R den Rest Chinuclidinyl-3 oder -4 darstellt,unter seinen isomeren Formen und deren Gemischen, sowie seine pharmazeutisch annehmbaren Salze.

2. Das 5δ-[Chinuclidinyl-3(S)]-thiomethyl-pristinamycin $I_A$ sowie seine pharmazeutisch annehmbaren Salze.

3. Das 5δ-[Chinuclidinyl-3(R)]-thiomethyl-pristinamycin $I_A$ sowie seine pharmazeutisch annehmbaren Salze.

4. Das 5δ-[Chinuclidinyl-3(S)]-thiomethyl-virginiamycin S sowie seine pharmazeutisch annehmbaren Salze.

5. Das 5δ-[Chinuclidinyl-3(R)]-thiomethyl-virginiamycin S sowie seine pharmazeutisch annehmbaren Salze.

6. Verfahren zur Herstellung eines Produktes gemäß Anspruch 1, dadurch gekennzeichnet, daß man ein Thiol der allgemeinen Formel

R-SH (II)

worin R wie in Anspruch 1 definiert ist, auf ein Produkt der allgemeinen Formel (III)

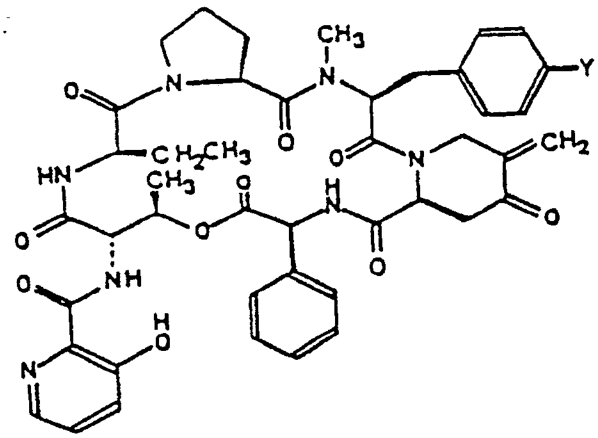

(III)

worin Y wie in Anspruch 1 definiert ist, einwirken läßt, dann das Produkt isoliert, gegebenenfalls seine Isomeren trennt und es gegebenenfalls in ein pharmazeutisch annehmbares Salz überführt.

7. Pharmazeutische Zusammensetzung, dadurch gekennzeichnet, daß sie wenigstens ein Derivat gemäß Anspruch 1 in reinem Zustand oder zusammen mit einem oder mehreren verträglichen und pharmazeutisch annehmbaren Verdünnungs- oder Hilfsmitteln und/oder zusammen mit Pristinamycin $II_A$ oder einem löslichen Derivat des Pristinamycins $II_B$ der allgemeinen Formel (VII)

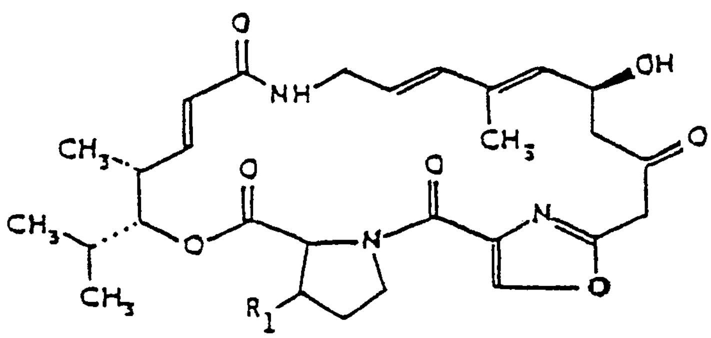

(VII)

enthält, worin $R_1$ bedeutet

1. einen Alkylthiorest mit 1 bis 5 Kohlenstoffatomen substituiert
   - i) durch einen oder zwei Alkylamino- oder Dialkylaminoreste, deren Alkylteile mit 1 bis 5 Kohlenstoffatomen gegebenenfalls zusammen mit dem Stickstoffatom, an das sie gebunden sind, einen gesättigten Heterocyclus bilden können, ausgewählt unter Pyrrolidinyl-1, Piperidino, Azetidinyl-1, Azepinyl-1, Morpholino, Thiomorpholino und Piperazinyl-1 (gegebenenfalls substituiert durch einen Alkylrest mit 1 bis 5 Kohlenstoffatomen) oder
   - ii) durch einen Rest Pyrrolidinyl-2 oder -3, Piperidyl-2, -3 oder -4, Azetidinyl-2 oder -3 oder Azepinyl-2, -3 oder -4,
2. einen Rest der allgemeinen Formel

   Het-S-

   worin, Het einen Rest Pyrrolidinyl-3, Piperidyl-3 oder -4, Azetidinyl-3 oder Azepinyl-3 oder -4, gegebenenfalls substituiert durch einen Alkylrest mit 1 bis 5 Kohlenstoffatomen bedeutet,
3. einen Dialkylamino-Rest, dessen Alkylteile gegebenenfalls zusammen mit dem Stickstoffatom, an das sie gebunden sind, einen gesättigten Heterozyklus bilden können, ausgewählt unter Pyrrolidinyl-1, Piperidino, Azetidinyl-1, Azepinyl-1, Morpholino, Thiomorpholino und Piperazinyl-1 (gegebenenfalls substituiert durch einen Alkylrest mit 1 bis 5 Kohlenstoffatomen),
4. einen Rest der allgemeinen Formel

$$R_2 - \underset{(O)_n}{S} -$$

worin $R_2$ bedeutet

- entweder einen Stickstoff-Heterocyclyl-Rest mit 4 bis 7 Kettengliedern enthaltend gegebenenfalls ein oder mehrere weitere Heteroatome ausgewählt unter Stickstoff, Sauerstoff oder Schwefel im Zustand des Sulfoxids oder Sulfons und gegebenenfalls substituiert durch einen Alkylrest,
- oder eine Alkylkette enthaltend 2 bis 4 Kohlenstoffatome substituiert durch einen oder zwei Reste ausgewählt unter Phenyl, Cycloalkylamino oder N-Alkyl-N-cycloalkylamino enthaltend 3 bis 6 Kettenglieder, Alkylamino, Dialkylamino oder Dialkylcarbamoyloxy (die Alkylteile dieser zwei letzteren Reste können gegebenenfalls mit dem Stickstoffatom, an das sie gebunden sind, einen gesättigten oder ungesättigten Heterocyclus mit 4 bis 7 Kettengliedern bilden, enthaltend gegebenenfalls ein weiteres Heteroatom ausgewählt unter Stickstoff, Sauerstoff oder Schwefel im Zustand des Sulfoxids oder Sulfons und gegebenenfalls substituiert durch einen Alkylrest) oder substituiert durch einen oder mehrere Stickstoffheterozyklen mit 4 bis 7 Kettengliedern enthaltend gegebenenfalls ein oder zwei weitere Heteroatome ausgewählt unter Stickstoff, Sauerstoff oder Schwefel im Zustand des Sulfoxids oder Sulfons und gegebenenfalls substituiert durch einen Alkylrest, wobei diese Heterozyklen an die sie tragende Kette gebunden sind über ein Kohlenstoffatom, wobei wenigstens einer der Substituenten, der durch die obige Alkylkette getragen ist, ein Stickstoffsubstituent ist, der Salze zu bilden vermag, oder einen [1-Methylpyrrolidinyl-2-(S)]-methyl-Rest, wobei n = 1 oder 2, wobei die oben erwähnten Alkylreste gerade oder verzweigt sind und, wenn nichts anderes angegeben ist, 1 bis 10 Kohlenstoffatome enthalten, gegebenenfalls in Form eines seiner Isomeren oder deren Gemische und gegebenenfalls in Form des Additionssalzes mit einer Säure.

Patentanspruch für folgende Vertragsstaaten : AT, ES, GR

1. Verfahren zur Herstellung eines neuen Synergistinderivats der allgemeinen Formel:

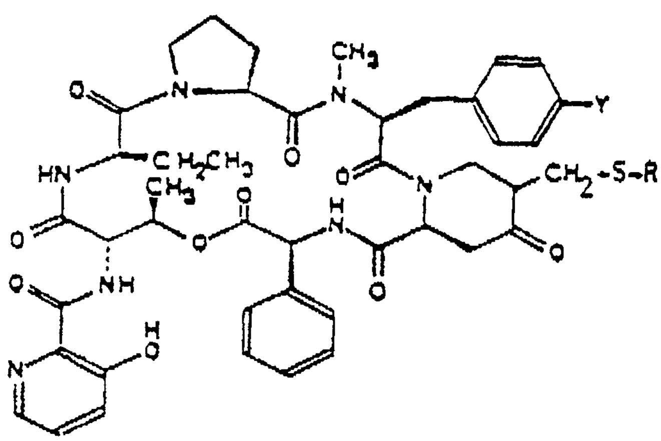

(I)

in welcher Y ein wasserstoffatom oder einen Dimethylaminorest darstellt und R den 3- oder 4-Chinuclidinylrest darstellt, dadurch gekennzeichnet, daß man ein Thiol der allgemeinen Formel:

R-SH

in welcher R die obige Bedeutung hat, mit einer Verbindung der allgemeinen Formel:

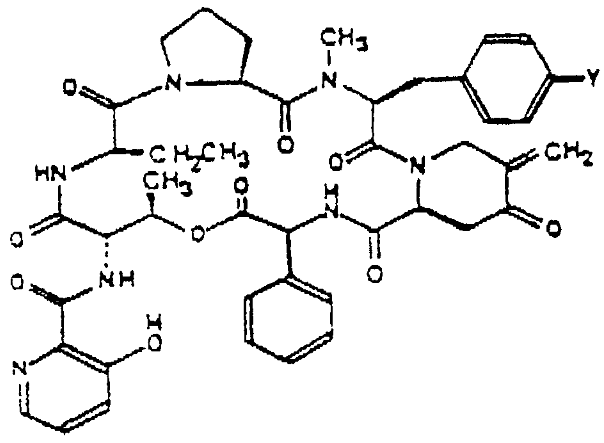

umsetzt, in welcher Y die obige Bedeutung hat, dann das Produkt isoliert, gegebenenfalls seine Isomeren trennt und es gegebenenfalls in ein pharmazeutisch zulässiges Salz umwandelt.